# EUROPÄISCHE PATENTSCHRIFT

(11) **EP 4 478 046 B1**
(45) Veröffentlichungstag und Bekanntmachung des Hinweises auf die Patenterteilung: **28.10.2026**
(21) Anmeldenummer: 24176492.7
(22) Anmeldetag: 17.05.2024
(51) Int. Cl.: G01N 33/00

(54) **GASMESSGERÄT MIT AUTOMATISCHER ERKENNUNG DER KONFIGURATION UND KONFIGURIERUNGS-VERFAHREN**
GAS METER WITH AUTOMATIC CONFIGURATION RECOGNITION AND CONFIGURATION METHOD
COMPTEUR DE GAZ AVEC DÉTECTION AUTOMATIQUE DE CONFIGURATION ET PROCÉDÉ DE CONFIGURATION

(30) Priorität: 26.05.2023 DE 102023113906
(43) Veröffentlichungstag der Anmeldung: 18.12.2024
(73) Patentinhaber: Dräger Safety AG & Co. KGaA, 23560 Lübeck (DE)
(72) Erfinder: Sturm, Hannes, 23558 Lübeck (DE); Hansen, Florian, 23558 Lübeck (DE); Wilhelm, Christian, 23558 Lübeck (DE); Einecke, Kai, 23558 Lübeck (DE); Martens, Matthias, 23558 Lübeck (DE)

(56) Entgegenhaltungen:
- EP-A1- 0 472 131
- WO-A1-01/14873
- WO-A1-2020/225796
- US-A1- 2002 178 789
- US-A1- 2015 177 207
- US-A1- 2018 267 003
- US-A1- 2023 113 553

## Beschreibung

Die Erfindung betrifft eine konfigurierbare Anordnung umfassend ein Gasmessgerät, wobei die Anordnung automatisch ihre eigene aktuelle Konfiguration zu erkennen vermag, sowie ein Konfigurierungs-Verfahren zum Konfigurieren einer derartigen Anordnung.

Die in US 2018 / 0 267 003 A1 beschriebene Anordnung umfasst ein Gasmessgerät, Erweiterungsmodule mit NFC-Datenspeichern, eine Auswerteeinheit und ein Lesegerät. Das Gasmessgerät ist dazu ausgestaltet, in den NFC-Datenspeichern gespeicherte Instruktionen berührungslos auszulesen und abhängig davon einen Vorgang auszulösen.

Der Erfindung liegt die Aufgabe zugrunde, eine Anordnung umfassend ein Gasmessgerät und mindestens ein Erweiterungsmodul bereitzustellen, wobei die Anordnung sich in unterschiedlichen Konfigurationen einsetzen lässt und wobei die Betriebssicherheit des Gasmessgeräts verglichen mit bekannten Anordnungen vergrößert ist. Weiterhin liegt der Erfindung die Aufgabe zugrunde, ein Verfahren zum Konfigurieren einer derartigen Anordnung bereitzustellen.

Die Aufgabe wird durch eine Anordnung mit den Merkmalen des Anspruchs 1 und durch ein Konfigurierungs-Verfahren mit den Merkmalen des Anspruchs 6 gelöst. Vorteilhafte Ausgestaltungen der Anordnung werden in den Unteransprüchen spezifiziert. Vorteilhafte Ausgestaltungen der erfindungsgemäßen Anordnung sind, soweit sinnvoll, auch vorteilhafte Ausgestaltungen des erfindungsgemäßen Konfigurierungs-Verfahrens und umgekehrt.

Die erfindungsgemäße Anordnung umfasst ein Gasmessgerät, welches sich auch als Gasdetektionsgerät oder Analysegerät bezeichnen lässt. Das Gasmessgerät ist dazu ausgestaltet, in einer Gasprobe mindestens ein vorgegebenes Gas zu detektieren, insbesondere ein brennbares und / oder für Menschen toxisches Gas. Ein zu detektierendes Gas wird als ein "Zielgas" bezeichnet. Die Gasprobe stammt aus einem zu überwachenden räumlichen Bereich, beispielsweise aus einem Gebäude oder Fahrzeug oder einem Bereich einer Produktionsanlage oder Lagerfläche.

Das Gasmessgerät umfasst ein Basisteil, mindestens einen Gassensor, eine signalverarbeitende Auswerteeinheit und mindestens ein Lesegerät. Bevorzugt nimmt das Basisteil den Gassensor und eine Messkammer für die Gasprobe auf.

Der oder jeder Gassensor vermag jeweils mindestens eine Detektionsgröße zu messen, insbesondere eine elektrische Detektionsgröße. Die oder jede Detektionsgröße korreliert mit der jeweiligen Konzentration des oder mindestens eines zu detektierenden Zielgases. In der Regel ist die Detektionsgröße umso größer, manchmal aber umso kleiner, je größer die Zielgas-Konzentration ist. In einer Ausgestaltung korreliert die Detektionsgröße mit der Summe von mehreren Zielgas-Konzentrationen.

Die Auswerteeinheit vermag die Konzentration des oder mindestens eines zu detektierenden Zielgases wenigstens näherungsweise zu ermitteln. Optional ermittelt die Auswerteeinheit die summierte Konzentration von mehreren Zielgasen. Um die Zielgas-Konzentration zu ermitteln, verwendet die Auswerteeinheit mindestens einen gemessenen Wert der oder mindestens einer Detektionsgröße, optional einen zeitlichen Verlauf der Detektionsgröße. Bevorzugt wendet die Auswerteeinheit eine vorgegebene rechnerauswertbare Rechenvorschrift auf die Detektionsgröße an, um die Zielgas-Konzentration zu ermitteln.

Die erfindungsgemäße Anordnung umfasst mindestens ein Erweiterungsmodul, optional mehrere Erweiterungsmodule. Das oder jedes Erweiterungsmodul lässt sich lösbar mit dem Basisteil verbinden, also mit dem Basisteil verbinden und auch wieder vom Basisteil lösen. Die Verbindung ist bevorzugt eine mechanische Verbindung. Falls ein Erweiterungsmodul mit dem Basisteil verbunden ist, ist eine mögliche Bewegung des Erweiterungsmoduls relativ zum Basisteil begrenzt. In einer Realisierungsform kann das verbundene Erweiterungsmodul überhaupt keine Bewegung relativ zum Basisteil ausführen, die größer als eine Toleranz ist. Falls das Erweiterungsmodul vom Basisteil gelöst ist, kann das Erweiterungsmodul in der Regel relativ zum Basisteil beliebig bewegt werden. Die Begriffe "mit dem Basisteil verbinden" und "vom Basisteil lösen" können zusätzlich zu einer mechanischen Verbindung den Vorgang umfassen, eine Datenverbindung zwischen dem Basisteil und dem Erweiterungsmodul herzustellen bzw. diese Datenverbindung wieder zu unterbrechen oder zu beenden. Möglich ist auch, dass das Basisteil ein verbundenes Erweiterungsmodul mit elektrischer Energie versorgt.

**In** einer Ausgestaltung umfasst die Anordnung mehrere Erweiterungsmodule. **In** einer Realisierungsform lässt sich zu einem Zeitpunkt nur jeweils ein Erweiterungsmodul mit dem Basisteil verbinden. In einer anderen Ausgestaltung lassen sich mehrere Erweiterungsmodule gleichzeitig mit dem Basisteil verbinden.

Wie bereits erwähnt, umfasst das Gasmessgerät mindestens einen Gassensor. Der oder jeder Gassensor vermag jeweils eine Detektionsgröße zu messen, welche mit einer Zielgas-Konzentration korreliert. Der oder jeder Gassensor gehört zum Gasmessgerät und vermag die Detektionsgröße zu messen, und zwar unabhängig davon, ob das oder mindestens ein Erweiterungsmodul der erfindungsgemäßen Anordnung mit dem Basisteil verbunden ist oder das oder jedes Erweiterungsmodul vom Basisteil gelöst ist.

Das oder mindestens ein Erweiterungsmodul, bevorzugt jedes Erweiterungsmodul, umfasst jeweils einen Erweiterungsmodul-Datenspeicher. In diesem Erweiterungsmodul-Datenspeicher ist eine Kennzeichnung des Erweiterungsmoduls abgespeichert, und zwar in einer rechnerauswertbaren Form. Diese Kennzeichnung unterscheidet dieses Erweiterungsmodul von jedem anderen oder wenigstens von jedem andersartigen Erweiterungsmodul, das sich mit dem Basisteil lösbar verbinden lässt. In einer Ausgestaltung unterscheidet die Kennzeichnung das Erweiterungsmodul von dem oder jedem anderen Erweiterungsmodul der Anordnung. Die Kennzeichnung umfasst beispielsweise eine Sachnummer, die den Typ des Erweiterungsmoduls kennzeichnet, und eine Seriennummer, die dieses Erweiterungsmodul von jedem gleichartigen Erweiterungsmodul unterscheidet.

Das Gasmessgerät umfasst mindestens ein Lesegerät. Bevorzugt ist das oder jedes Lesegerät in das Basisteil integriert. Das oder jedes Lesegerät vermag den Erweiterungsmodul-Datenspeicher eines Erweiterungsmoduls auszulesen, wenn das Erweiterungsmodul mit dem Basisteil verbunden ist. Erfindungsgemäß vermag das Lesegerät den Erweiterungsmodul-Datenspeicher berührungslos auszulesen, also auch dann auszulesen, wenn ein Abstand zwischen dem Lesegerät und dem Erweiterungsmodul-Datenspeicher auftritt. Das Lesegerät vermag ein Signal zu erzeugen, wobei das Signal ein Ergebnis vom Auslesen des Erweiterungsmodul-Datenspeichers umfasst. Bevorzugt umfasst das Signal entweder eine ausgelesene Kennzeichnung eines Erweiterungsmoduls oder eine Codierung dafür, dass das Lesegerät kein Erweiterungsmodul erkannt hat oder den Erweiterungsmodul-Datenspeicher und daher die Kennzeichnung nicht auslesen konnte.

Das Gasmessgerät vermag automatisch festzustellen, ob das oder mindestens ein Erweiterungsmodul, welches einen Erweiterungsmodul-Datenspeicher umfasst, aktuell mit dem Basisteil verbunden ist oder nicht. Hierfür verarbeitet das Gasmessgerät das Signal des oder mindestens eines Lesegeräts. Möglich ist, dass das Gasmessgerät mehrere Lesegeräte umfasst und das jeweilige Signal von jedem Lesegerät verarbeitet.

Falls das Gasmessgerät festgestellt hat, dass mindestens ein solches Erweiterungsmodul mit dem Basisteil verbunden ist, so vermag das Gasmessgerät wie folgt zu reagieren:
- Das Gasmessgerät vermag durch Auswertung des Signals die abgespeicherte Kennzeichnung eines mit dem Basisteil verbundenen Erweiterungsmoduls zu ermitteln.
- Das Gasmessgerät löst automatisch mindestens einen Vorgang aus. Das Auslösen und / oder die Art dieses Vorgangs hängt von dem Vorhandensein und / oder von der Art der im Erweiterungsmodul-Datenspeicher abgespeicherten und ermittelten Kennzeichnung ab.
- Der ausgelöste Vorgang ist der folgende Vorgang: Die Auswerteeinheit wendet bei Vorhandensein des Erweiterungsmoduls eine andere Rechenvorschrift auf die Detektionsgröße an, um die Zielgas-Konzentration zu ermitteln, als bei Abwesenheit des Erweiterungsmoduls.

Das erfindungsgemäße Konfigurierungs-Verfahren bezieht sich auf eine solche Anordnung und umfasst die folgenden Schritte:
- Das oder ein Erweiterungsmodul wird lösbar mit dem Basisteil verbunden. Dieses Erweiterungsmodul umfasst einen Erweiterungsmodul-Datenspeicher, in welchem eine Kennzeichnung des Erweiterungsmoduls abgespeichert ist.
- Das oder ein Lesegerät des Gasmessgeräts liest berührungslos den Erweiterungsmodul-Datenspeicher dieses Erweiterungsmoduls aus.
- Das Gasmessgerät stellt automatisch fest, dass dieses Erweiterungsmodul nunmehr mit dem Basisteil verbunden ist. Hierfür verwendet das Gasmessgerät ein Signal des Lesegeräts.
- Das Gasmessgerät ermittelt die im Erweiterungsmodul-Datenspeicher abgespeicherte und vom Lesegerät eingelesene Kennzeichnung.
- Das Gasmessgerät löst einen Vorgang aus, und zwar abhängig von dem Vorhandensein und / oder der Art der Kennzeichnung, welche im Erweiterungsmodul-Datenspeicher abgespeichert ist und welche das Gasmessgerät ermittelt hat.

Die Erfindung ermöglicht es, dasselbe Basisteil und damit denselben Gassensor eines Gasmessgeräts in Verbindung mit unterschiedlichen Erweiterungsmodulen und damit in unterschiedlichen Konfigurationen zu betreiben. Insbesondere lässt sich das Gasmessgerät durch die unterschiedlichen Konfigurationen an verschiedene Aufgaben und / oder Anwendungen anpassen. Dank der Erfindung ist es oft nicht erforderlich, für jede Anwendung und / oder für jede Aufgabe jeweils ein eigenes Gasmessgerät bereitzuhalten. Dadurch werden Bauteile eingespart.

Erfindungsgemäß liest das oder ein Lesegerät des Gasmessgeräts eine Kennzeichnung ein, die auf einem Datenspeicher eines Erweiterungsmoduls abgespeichert ist. Weil das Lesegerät die Kennzeichnung berührungslos einliest, ist es in vielen Fällen nicht erforderlich, das Erweiterungsmodul exakt positionsrichtig relativ zum Lesegerät oder zu einem sonstigen Sensor, beispielsweise einem Kontaktschalter, des Basisteils zu positionieren. Dank dieses Merkmals wird die Gefahr reduziert, dass fälschlich ein verbundenes Erweiterungsmodul nicht detektiert wird oder ein falsches Erweiterungsmodul erkannt wird. Außerdem ist die Gefahr geringer als bei einer Kennzeichnung, die sich optisch einlesen lässt, dass sich die Kennzeichnung aufgrund einer Verschmutzung oder Beschädigung nicht korrekt ermitteln lässt.

Das Gasmessgerät vermag automatisch auf die eingelesene Kennzeichnung zu reagieren und hierbei festzustellen, dass nunmehr das betreffende Erweiterungsmodul mit dem Basisteil verbunden ist. Dank der Erfindung ist es nicht erforderlich, dass ein Benutzer einen Benutzereingriff vornimmt, um dem Gasmessgerät "mitzuteilen", dass und welches Erweiterungsmodul oder welche Erweiterungsmodule aktuell mit dem Basisteil verbunden ist / sind. Dadurch wird die Gefahr reduziert, dass das Gasmessgerät aufgrund eines Benutzerfehlers ein falsches Messergebnis liefert. Außerdem wird in vielen Fällen Zeit eingespart. Umgekehrt vermag das Gasmessgerät automatisch festzustellen, dass es aktuell nicht mit einem oder einem bestimmten Erweiterungsmodul verbunden ist.

Erfindungsgemäß vermag das Gasmessgerät abhängig von dem Signal des Lesegeräts festzustellen, ob das Erweiterungsmodul mit dem Basisteil verbunden ist oder nicht. Bevorzugt umfasst das Gasmessgerät ein Programm, das in einem Datenspeicher abgespeichert ist und bei einem Einsatz des Gasmessgeräts von einem Prozessor ausgeführt wird. Dieses Programm wertet die oder jede eingelesene Kennzeichnung aus und löst eine Reaktion des Gasmessgeräts aus, wobei diese Reaktion von der eingelesenen Kennzeichnung abhängt. Erfindungsgemäß ermittelt das oder ein Lesegerät oder auch ein Auswerteprogramm die Kennzeichnung eines Erweiterungsmoduls, wobei diese Kennzeichnung im Erweiterungsmodul-Datenspeicher abgespeichert ist und das Lesegerät diese Kennzeichnung einliest. Das Gasmessgerät wertet automatisch die eingelesene Kennzeichnung aus und reagiert auf die eingelesene Kennzeichnung, indem das Gasmessgerät einen Vorgang durchführt, der von dem Vorhandensein und / oder der Art der Kennzeichnung abhängt.

Erfindungsgemäß vermag das Gasmessgerät automatisch auf das Ereignis zu reagieren, dass das Lesegerät von einem Erweiterungsmodul-Datenspeicher die Kennzeichnung dieses Erweiterungsmoduls eingelesen hat. Unterschiedliche Ausgestaltungen sind möglich, wie das Gasmessgerät auf dieses Ereignis reagiert. Bevorzugt hängt die Reaktion auf das Ereignis davon ab, welche Kennzeichnung eingelesen wurde. Besonders bevorzugt sind unterschiedliche Mengen von Kennzeichnungen definiert, und die Reaktion hängt davon ab, zu welcher der vorgegebenen Mengen die eingelesene Kennzeichnung gehört. Beispielsweise ist oder umfasst jede mögliche Kennzeichnung eines Erweiterungsmoduls jeweils eine Sachnummer und eine Seriennummer, wobei alle Erweiterungsmodule mit derselben Sachnummer baugleich sind und sich idealerweise nur durch die jeweilige Seriennummer voneinander unterscheiden. Die Reaktion des Gasmessgeräts hängt mindestens von der eingelesenen Sachnummer ab, optional zusätzlich von der eingelesenen Seriennummer.

Erfindungsgemäß misst der Gassensor eine Detektionsgröße. Die Auswerteeinheit verwendet mindestens einen gemessenen Wert der Detektionsgröße, um die Zielgas-Konzentration zu ermitteln. In der Regel wendet die Auswerteeinheit einen funktionalen Zusammenhang zwischen der Detektionsgröße und der Zielgas-Konzentration auf den gemessenen Detektionsgrößen-Wert oder Detektionsgrößen-Verlauf an, um die Zielgas-Konzentration herzuleiten. Im einfachsten Fall ist der funktionale Zusammenhang ein Proportionalitätsfaktor oder allgemeiner eine Kennlinie.

Erfindungsgemäß vermag der Gassensor des Gasmessgeräts eine Detektionsgröße zu messen. Diese Detektionsgröße korreliert mit der Konzentration mindestens eines Zielgases. Erfindungsgemäß sind in einem Auswertungs-Datenspeicher des Gasmessgeräts mindestens zwei verschiedene funktionale Zusammenhänge zwischen derselben Detektionsgröße und der Konzentration desselben Zielgases abgespeichert. Die Auswerteeinheit oder auch ein signalverarbeitendes Steuergerät des Gasmessgeräts wählt automatisch einen funktionalen Zusammenhang aus und wendet diesen auf den Detektionsgrößen-Wert oder Detektionsgrößen-Verlauf an. Falls ein Erweiterungsmodul eines bestimmten Typs mit dem Basisteil verbunden ist, wird ein erster funktionaler Zusammenhang angewendet. Falls dieses Erweiterungsmodul nicht mit dem Basisteil verbunden ist, wird ein weiterer funktionaler Zusammenhang angewendet. Die beiden funktionalen Zusammenhänge unterscheiden sich voneinander. Die Verbindung dieses Erweiterungsmoduls mit dem Basisteil verändert also eine Abhängigkeit der Detektionsgröße von der Zielgas-Konzentration. Der erste Zusammenhang ist gültig und wird angewendet, falls dieses Erweiterungsmodul mit dem Basisteil verbunden ist, der weitere funktionale Zusammenhang, falls das Erweiterungsmodul nicht mit dem Basisteil verbunden ist.

**In** einer Fortbildung sind drei verschiedene funktionale Zusammenhänge vorgegeben, und die Anordnung umfasst zwei verschiedene Erweiterungsmodule. Falls kein Erweiterungsmodul mit dem Basisteil verbunden ist, wird ein erster funktionaler Zusammenhang angewendet, falls das erste Erweiterungsmodul verbunden ist, ein zweiter funktionaler Zusammenhang, und falls das zweite Erweiterungsmodul verbunden ist, ein dritter funktionaler Zusammenhang.

Weiter oben wurde beschrieben, dass die Auswerteeinheit einen ersten oder einen zweiten funktionalen Zusammenhang auf die Detektionsgröße anwendet, je nachdem ob ein Erweiterungsmodul mit dem Basisteil verbunden ist oder nicht. Dieses Merkmal ermöglicht es in vielen Fällen, den Einfluss zu berücksichtigen und bis zu einem gewissen Grad zu kompensieren, den das Erweiterungsmodul auf die Detektionsgröße nimmt. Eine mögliche Anwendung: Das Erweiterungsmodul verändert die chemische Zusammensetzung und / oder eine physikalische Eigenschaft einer Gasprobe, die aus einem zu überwachenden räumlichen Bereich in eine Messkammer des Gasmessgeräts fließt und ein Zielgas enthält oder enthalten kann. Beispielsweise absorbiert oder verändert das Erweiterungsmodul einen Teil eines Zielgases.

In einer Ausgestaltung hängt die Detektionsgröße von der Zielgas-Konzentration und zusätzlich von einer Umgebungsbedingung ab, insbesondere von der Umgebungstemperatur und / oder der Umgebungsfeuchte und / oder dem Umgebungsdruck. Diese Umgebungsbedingung tritt im ersten funktionalen Zusammenhang auf, während im weiteren funktionalen Zusammenhang ein Standardwert für diese Umgebungsbedingung auftritt. Das oder ein Erweiterungsmodul umfasst einen Sensor für diese Umgebungsbedingung. Möglich ist auch, dass das Erweiterungsmodul einen Empfänger umfasst, wobei der Empfänger dazu ausgestaltet ist, ein Signal von einem räumlich entfernten Sensor für die Umgebungsbedingung zu empfangen.

Falls das Gasmessgerät feststellt, dass das Erweiterungsmodul mit diesem Umgebungsbedingungs-Sensor oder mit diesem Empfänger mit dem Basisteil verbunden ist, so wendet die Auswerteeinheit den ersten funktionalen Zusammenhang an, ansonsten den weiteren funktionalen Zusammenhang. Das Gasmessgerät verwendet einen Signalwert vom Sensor für die Umgebungsbedingung und den ersten funktionalen Zusammenhang, um eine Zielgas-Konzentration zu ermitteln.

Diese Ausgestaltung ermöglicht es in vielen Fällen, dann den Einfluss der Umgebungsbedingung relativ genau zu berücksichtigen, wenn das Erweiterungsmodul mit dem Sensor diese Umgebungsbedingung misst oder empfängt, und ansonsten einen Standardwert zu verwenden. Dank dieser Ausgestaltung mit dem Umgebungsbedingungs-Sensor lässt sich dasselbe Basisteil sowohl in Verbindung mit dem Sensor als auch ohne den Sensor verwenden. Nicht erforderlich ist, dass ein Benutzer eine Vorgabe treffen muss, um vorzugeben, ob ein Sensor für die Umgebungsbedingung mit dem Basisteil verbunden ist oder nicht.

In einer Ausgestaltung, die nicht unter den Anspruchsgegenstand fällt, nimmt das oder ein Erweiterungsmodul Einfluss auf die Zeitdauer, die eine Gasprobe benötigt, bis die Gasprobe den oder einen Gassensor des Gasmessgeräts erreicht. Diese Gasprobe stammt aus einer Umgebung des Gasmessgeräts. Insbesondere verändert gemäß dieser Ausgestaltung das Erweiterungsmodul die Wegstrecke, über die eine Gasprobe fließen muss, bis sie eine Messkammer und damit den oder einen Gassensor erreicht. Eine mögliche Realisierungsform dieses Erweiterungsmoduls umfasst einen Schlauch oder eine Röhre oder eine sonstige Fluidführungseinheit, die sich lösbar und fluiddicht mit dem Basisteil verbinden lässt. Diese Fluidführungseinheit hat zwei Enden, wobei bei montierter Fluidführungseinheit das eine Ende eine Öffnung des Basisteils fluiddicht überdeckt und das andere Ende mit einem räumlichen Bereich verbunden ist, der auf das Vorhandensein eines Zielgases zu überwachen ist.

Falls dieses Erweiterungsmodul mit dem Basisteil verbunden ist, so wird Folgendes bewirkt: Eine Gasprobe aus dem räumlichen Bereich fließt durch die Fluidführungseinheit und die Öffnung im Basisteil hindurch in das Innere des Basisteils und kann nicht direkt, also unter Umgehung der Fluidführungseinheit, in das Innere und damit zum Gassensor gelangen. Falls das Erweiterungsmodul nicht mit dem Basisteil verbunden ist, so kann die Gasprobe direkt durch die Öffnung hindurch in das Innere gelangen, also ohne durch die Fluidführungseinheit hindurch fließen zu müssen.

Dank der Fluidführungseinheit ist es nicht erforderlich, das Basisteil in dem zu überwachenden räumlichen Bereich anzubringen. Die fluiddicht verbundene Fluidführungseinheit verhindert, dass Gas aus der unmittelbaren Umgebung des Gasmessgeräts zum Gassensor gelangt. Falls die Fluidführungseinheit nicht mit dem Basisteil verbunden ist, gelangt die Gasprobe aus der Umgebung direkt durch die Öffnung hindurch in das Innere des Basisteils und damit zum Gassensor.

Gemäß dieser Ausgestaltung verlängert das mit dem Basisteil verbundene Erweiterungsmodul die Zeitdauer, die verstreicht, bis eine Gasprobe aus dem räumlichen Bereich den Gassensor erreicht, verglichen mit einer Ausgestaltung ohne dieses Erweiterungsmodul. Die Auswerteeinheit berücksichtigt diese Zeitdauer beim Schritt, abhängig von der Detektionsgröße, bevorzugt abhängig vom zeitlichen Verlauf der Detektionsgröße, die Zielgas-Konzentration zu ermitteln. Insbesondere ermöglicht diese Ausgestaltung es, eine Warnung oder einen Alarm betreffend das Vorhandensein eines Zielgases und / oder eine Meldung über das Fehlen des Zielgases so früh wie möglich zu generieren. Andererseits stellt diese Ausgestaltung in vielen Fällen sicher, dass eine Mitteilung, dass kein zu detektierendes Zielgas vorhanden ist, erst dann ausgegeben wird, wenn eine Gasprobe aus dem zu überwachenden Bereich eine Messkammer im Inneren des Basisteils erreicht hat. Dadurch ist die Gefahr relativ gering, dass trotz des Vorhandenseins eines Zielgases weder eine Warnung noch ein Alarm generiert werden.

Erfindungsgemäß umfasst das Gasmessgerät ein Basisteil und mindestens einen Gassensor. Bevorzugt ist der oder jeder Gassensor des Gasmessgeräts fest mit dem Basisteil verbunden. Daher ist es nicht erforderlich, mit Hilfe eines Lesegeräts zu überprüfen, ob ein Gassensor des Gasmessgeräts mit dem Basisteil verbunden ist oder nicht.

In einer Ausgestaltung umfasst das oder mindestens ein Erweiterungsmodul einen weiteren Gassensor. Auch der weitere Gassensor vermag eine Detektionsgröße zu messen, welche mit der Konzentration eines vorgegebenen Zielgases korreliert. Möglich ist, dass sich sowohl die Detektionsgröße, die der Gassensor des Gasmessgeräts zu messen vermag, als auch die Detektionsgröße, die der weitere Gassensor des Erweiterungsmoduls zu messen vermag, auf dasselbe Zielgas beziehen. In vielen Fällen erhöht dies die Zuverlässigkeit, dass ein Zielgas tatsächlich detektiert wird, nämlich von wenigstens einem Gassensor.

Eine Kennzeichnung des weiteren Gassensors ist im Datenspeicher dieses Erweiterungsmoduls abgespeichert und wird vom Lesegerät eingelesen. Bevorzugt vermag die Auswerteeinheit zusätzlich einen Detektionsgrößen-Wert zu verwenden, den der weitere Gassensor, also der Gassensor des Erweiterungsmoduls, gemessen hat.

In einer Realisierungsform vermag der weitere Gassensor eine andere Detektionsgröße zu messen als der Gassensor oder die Gassensoren des Gasmessgeräts. Dies erhöht den Anwendungsbereich der Anordnung. In einer anderen Ausgestaltung vermag der weitere Gassensor dieselbe Detektionsgröße zu messen wie der oder ein Gassensor des Gasmessgeräts. Dies führt in vielen Fällen zu gewollter Redundanz, insbesondere wenn die beiden Gassensoren unterschiedliche Sensorprinzipien anwenden. Möglich ist auch, dass sich der weitere Gassensor bei einer anderen Umgebungsbedingung, z.B. andere Temperatur oder Feuchte, als der Gassensor oder die Gassensoren des Gasmessgeräts einsetzen lässt. Der weitere Gassensor kann auch eine andere Lebensdauer und / oder eine andere Zuverlässigkeit aufweisen als der Gassensor oder die Gassensoren des Gasmessgeräts.

Die Ausgestaltung, dass der weitere Gassensor ein Bestandteil eines Erweiterungsmoduls ist, ermöglicht es, die Anordnung in einer Konfiguration von mehreren möglichen Konfigurationen zu verwenden. In einer ersten Konfiguration wird ausschließlich der oder jeder Gassensor des Gasmessgerät verwendet. In einer zweiten Konfiguration wird zusätzlich der weitere Gassensor des Erweiterungsmoduls verwendet.

Möglich ist, dass die Anordnung mindestens zwei verschiedene Erweiterungsmodule mit jeweils einem weiteren Gassensor umfasst. Die beiden weiteren Gassensoren, also die beiden Gassensoren der beiden Erweiterungsmodule, können für die Messung derselben Detektionsgröße ausgestaltet sein oder auch zur Messung von zwei verschiedenen Detektionsgrößen.

Bevorzugt lässt das Gasmessgerät sich einschalten und ausschalten. Dieses Merkmal spart bekanntlich elektrische Energie ein. In einer Ausgestaltung umfasst das oder mindestens ein Erweiterungsmodul ein Schutzelement, insbesondere eine Verschlusskappe. Falls dieses Schutzelement mit dem Basisteil verbunden ist, so trennt das Schutzelement einen Innenraum des Gasmessgeräts fluiddicht von der Umgebung. Falls das Schutzelement aufgesetzt ist, kann keine Gasprobe aus einem zu überwachenden räumlichen Bereich in das Innere des Gasmessgeräts fließen. In vielen Fällen wird bei verbundenem Schutzelement einerseits verhindert, dass schädliche Substanzen oder auch eine Reinigungsflüssigkeit oder Wassertröpfchen aus der Umgebung in das Innere des Basisteils und damit zu einem Gassensor im Basisteil gelangen. Andererseits wird bei verbundenem Schutzelement oft verhindert, dass ein Bestandteil eines Gassensors verdunstet, was ansonsten insbesondere bei einem elektrochemischen Gassensor passieren kann.

In einer Ausgestaltung, die nicht unter den Anspruchsgegenstand fällt, ist dieses Schutzelement ein Bestandteil eines Erweiterungsmoduls oder bildet ein Erweiterungsmodul. Falls das Gasmessgerät eingeschaltet ist, so vermag das Gasmessgerät dank der Erfindung automatisch festzustellen, ob das Schutzelement mit dem Basisteil verbunden ist oder nicht. Hierfür verwendet das Gasmessgerät ein Signal von dem oder einem Lesegerät, wobei dieses Lesegerät wiederum eine Kennzeichnung des Erweiterungsmoduls mit dem Schutzelement eingelesen hat. Falls bei eingeschaltetem Gasmessgerät das Schutzelement mit dem Basisteil verbunden ist, so generiert das Gasmessgerät einen Alarm. Das Gasmessgerät gibt diesen Alarm in mindestens einer von einem Menschen wahrnehmbaren Form aus oder veranlasst, dass eine externe Ausgabeeinheit diesen Alarm in einer von einem Menschen wahrnehmbaren Form ausgibt. Der Alarm wird insbesondere visuell und / oder akustisch und / oder haptisch (durch Vibrationen) ausgegeben.

Der Hintergrund: In der Regel wird das Gasmessgerät eingeschaltet, wenn es eine Gasprobe auf mindestens ein Zielgas untersuchen soll. Dies ist nur möglich, wenn die Gasprobe eine Messkammer und damit einen Gassensor im Inneren des Gasmessgeräts erreicht. Die Gasprobe kann nur dann die Messkammer erreichen, wenn das Schutzelement abgenommen ist. Falls also bei aufgesetztem Schutzelement das Gasmessgerät eingeschaltet wird, so ist dies ein Indiz für einen Fehler. Falls dieser Fehler nicht bemerkt wird, so besteht die Gefahr, dass ein Zielgas nicht detektiert wird. Diese Ausgestaltung reduziert also die Gefahr des unerwünschten Ereignisses, dass das Gasmessgerät mit aufgesetztem Schutzelement betrieben wird. In vielen Fällen erspart die Erfindung die Notwendigkeit, dass ein Mensch das Gasmessgerät darauf überprüfen muss, ob das Schutzelement aufgesetzt ist oder nicht. Dies ist insbesondere dann häufig mit Aufwand verbunden, wenn das Gasmessgerät stationär verwendet wird, also nicht von einem Menschen mit sich geführt wird. Außerdem kann ein Benutzer ein aufgesetztes Schutzelement übersehen. Weiterhin ist es möglich, aber dank der Ausgestaltung nicht erforderlich, einen Kontaktschalter vorzusehen, der das aufgesetzte Schutzelement detektiert.

In einer Ausgestaltung, die nicht unter den Anspruchsgegenstand fällt, umfasst das oder mindestens ein Erweiterungsmodul ein Kommunikationsmodul. Mithilfe dieses Kommunikationsmoduls lässt sich eine Datenverbindung, bevorzugt eine Datenverbindung per Funkwellen, zwischen dem Gasmessgerät und einem räumlich entfernten Empfänger herstellen. Diese Datenverbindung lässt sich gemäß der Ausgestaltung dann herstellen, wenn dieses Erweiterungsmodul mit dem Basisteil verbunden ist. Bevorzugt versorgt das Basisteil das verbundene Kommunikationsmodul mit elektrischer Energie. Das Gasmessgerät vermag eine Nachricht zu generieren und zu veranlassen, dass die Nachricht über die hergestellte Datenverbindung an den räumlich entfernten Empfänger übermittelt wird. Die Nachricht umfasst eine Kennzeichnung einer Zielgas-Konzentration, welche der Gassensor des Gasmessgeräts oder optional ein weiterer Gassensor eines Erweiterungsmoduls gemessen hat, und / oder eine Kennzeichnung eines Alarms, welchen das Gasmessgerät generiert hat.

Die Ausgestaltung mit dem Kommunikationsmodul als Teil eines Erweiterungsmoduls erspart die Notwendigkeit, das Gasmessgerät dauerhaft mit einem Kommunikationsmodul versehen zu müssen. Vielmehr ist es möglich, dasselbe Gasmessgerät einerseits dafür zu verwenden, um einen Benutzer zu warnen, der das Gasmessgerät mit sich führt. Das Gasmessgerät hat dann oft eine Ausgabeeinheit für einen Alarm. Häufig wird dann kein Kommunikationsmodul benötigt. Andererseits lässt sich das Gasmessgerät an einem Aufstellort aufstellen und übermittelt dann eine Nachricht mit der Zielgas-Konzentration an den räumlich entfernten Empfänger. Dafür wird ein Kommunikationsmodul benötigt.

In vielen Fällen soll ein Gasmessgerät in einer Umgebung eingesetzt werden, in der es sich nicht mit einem stationären Spannungsversorgungsnetz verbinden lässt. Daher umfasst das Gasmessgerät bevorzugt eine eigene Spannungsversorgungseinheit. Beispielsweise führt ein Benutzer das Gasmessgerät mit sich, während der Benutzer sich in einem räumlichen Bereich aufhält, in dem mindestens ein Zielgas auftreten kann. Bevorzugt besitzt das Gasmessgerät dann eine Ausgabeeinheit, welche den Benutzer bei einer hohen Zielgas-Konzentration warnen kann. Oder das Gasmessgerät wird an einem Ort aufgestellt, an dem ein Zielgas auftreten kann. Bevorzugt umfasst das Gasmessgerät dann eine Kommunikationseinheit, und über diese Kommunikationseinheit wird eine gemessene Zielgas-Konzentration und / oder ein Alarm an einen räumlich entfernten Empfänger übermittelt.

In einer Ausgestaltung umfasst das oder ein Erweiterungsmodul eine externe Spannungsversorgungseinheit. Diese externe Spannungsversorgungseinheit lässt sich elektrisch mit dem Gasmessgerät verbinden. Falls das Erweiterungsmodul umfassend die externe Spannungsversorgungseinheit mit dem Basisteil verbunden ist, so wird mindestens ein elektrischer Verbraucher des Gasmessgeräts wenigstens zeitweise aus dieser externen Spannungsversorgungseinheit heraus versorgt.

In manchen Fällen vermeidet diese Ausgestaltung die Notwendigkeit, das Gasmessgerät mit einer internen Spannungsversorgungseinheit versehen zu müssen. Vielmehr wird ermöglicht, das Gasmessgerät wahlweise mit einem stationären Spannungsversorgungsnetz oder mit der externen Spannungsversorgungseinheit zu verbinden. In manchen Fällen erhöht diese Ausgestaltung daher die Flexibilität, mit der sich das Gasmessgerät einsetzen lässt.

Nachfolgend wird eine andere Fortbildung der Ausgestaltung mit der externen Spannungsversorgungseinheit beschrieben. Das Gasmessgerät umfasst eine interne Spannungsversorgungseinheit. Diese befindet sich bevorzugt im Inneren des Basisteils. Mindestens ein elektrischer Verbraucher, bevorzugt jeder elektrische Verbraucher, lässt sich wahlweise aus der externen Spannungsversorgungseinheit des Erweiterungsmoduls oder aus der internen Spannungsversorgungseinheit versorgen - ersteres natürlich nur dann, wenn das Erweiterungsmodul mit dem Basisteil verbunden ist.

Bevorzugt bewirkt ein Steuergerät des Gasmessgeräts folgendes: Falls das Erweiterungsmodul umfassend die externe Spannungsversorgungseinheit mit dem Basisteil verbunden ist und der elektrische Füllstand der externen Spannungsversorgungseinheit ausreichend hoch ist, werden die elektrischen Verbraucher aus der externen Spannungsversorgungseinheit versorgt. Die interne Spannungsversorgungseinheit wird dann verwendet, wenn keine externe Spannungsversorgungseinheit mit dem Basisteil verbunden ist oder der Füllstand der externen Spannungsversorgungseinheit unter eine vorgegebene Schranke gefallen ist.

In vielen Fällen lässt sich eine externe Spannungsversorgungseinheit rascher austauschen als eine interne Spannungsversorgungseinheit. Daher ermöglicht die Ausgestaltung mit der externen Spannungsversorgungseinheit es, bei einem niedrigen Füllstand der internen oder der aktuell verbundenen externen Spannungsversorgungseinheit rasch eine neue externe Spannungsversorgungseinheit anzuschließen. Die ausgetauschte alte externe Spannungsversorgungseinheit lässt sich aufladen, während das Gasmessgerät mit der neuen externen Spannungsversorgungseinheit benutzt wird.

Die gerade beschriebene Ausgestaltung ist in manchen Fällen insbesondere dann wichtig, wenn das Gasmessgerät in einer explosionsgefährdeten Umgebung eingesetzt werden soll und daher als ein eigensicheres Gerät ausgestaltet ist. Häufig ist es zwar möglich, in der explosionsgefährdeten Umgebung eine externe Spannungsversorgungseinheit auszutauschen. Die interne Spannungsversorgungseinheit lässt sich hingegen nur außerhalb der explosionsgefährdeten Umgebung austauschen oder aufladen. Die gerade beschriebene Ausgestaltung vermeidet also die Notwendigkeit, das Gasmessgerät bei einem niedrigen Füllstand einer Spannungsversorgungseinheit aus der explosionsgefährdeten Umgebung zu tragen und später wieder in diese zu verbringen.

Im Folgenden wird die Erfindung anhand eines Ausführungsbeispiels beschrieben. Hierbei zeigt
- Figur 1: in einer perspektivischen Darstellung ein Gasmessgerät mit einer Anzeige- und Bedieneinheit;
- Figur 2: eine Anordnung umfassend ein Gasmessgerät mit einer internen Spannungsversorgungseinheit und einem Erweiterungsmodul mit einer externen Spannungsversorgungseinheit;
- Figur 3: in einer perspektivischen Darstellung das Gasmessgerät des Ausführungsbeispiels sowie eine Abdeckkappe für die Einlässe;
- Figur 4: in einer perspektivischen Darstellung ein Teil des Gasmessgeräts von Figur 3 sowie einen Adapter und einen Kalibrier-Adapter;
- Figur 5: das Gasmessgerät von Figur 3 und Figur 4 vor dem Aufsetzen des Kalibrier-Adapters;
- Figur 6: das Gasmessgerät von Figur 5 nach dem Aufsetzen des Kalibrier-Adapters;
- Figur 7: in einer Explosionsdarstellung ein Gasmessgerät mit verschiedenen Erweiterungsmodulen.

Im Ausführungsbeispiel wird das erfindungsgemäße Gasmessgerät dafür eingesetzt, um in einem räumlichen Bereich mindestens ein schädliches Zielgas zu detektieren, insbesondere ein brennbares oder toxisches Zielgas, oder sicherzustellen, dass in diesem räumlichen Bereich kein schädliches Zielgas vorhanden ist. Optional vermag das Gasmessgerät gleichzeitig mehrere schädliche Zielgase zu detektieren. Der räumliche Bereich kann insbesondere ein Raum sein, der vollständig oder wenigstens teilweise umschlossen ist und zu einem Gebäude oder einem Fahrzeug gehört, oder auch ein Bereich im Freien sein, beispielsweise eine Produktionsanlage oder Lagerfläche.

Falls in dem Bereich mehrere schädliche Zielgase vorhanden sind, so vermag das Gasmessgerät bevorzugt mindestens die Summe der Zielgas-Konzentrationen zu messen. In einer Ausgestaltung umfasst das Gasmessgerät mehrere Gassensoren und vermag daher die jeweilige Konzentration von mehreren schädlichen Zielgasen in dem räumlichen Bereich zu messen und / oder automatisch zu entscheiden, ob die Konzentration des oder eines Zielgases unterhalb oder oberhalb einer vorgegebenen Konzentrations-Schranke liegt. In einer Ausgestaltung vermag das Gasmessgerät mindestens eine gemessene Zielgas-Konzentration oder auch einen Alarm in einer von einem Menschen wahrnehmbaren Form auszugeben, also visuell und / oder akustisch und / oder haptisch (per Vibrationen). In einer anderen Ausgestaltung ist das Gasmessgerät dazu ausgestaltet, eine Zielgas-Konzentration oder einen Alarm an einen räumlich entfernten Empfänger zu übermitteln, der seinerseits die empfangene Nachricht ausgibt.

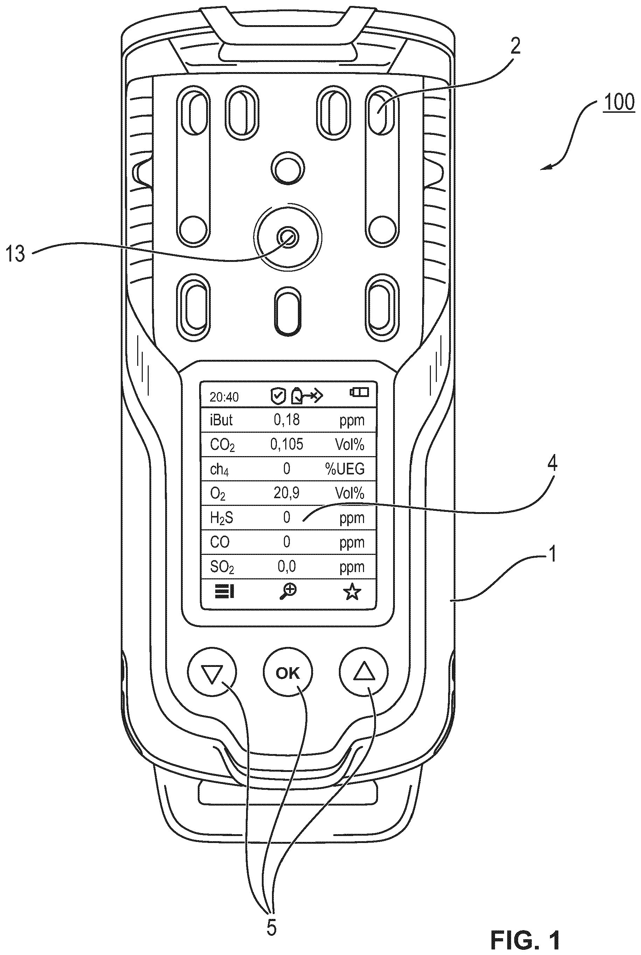

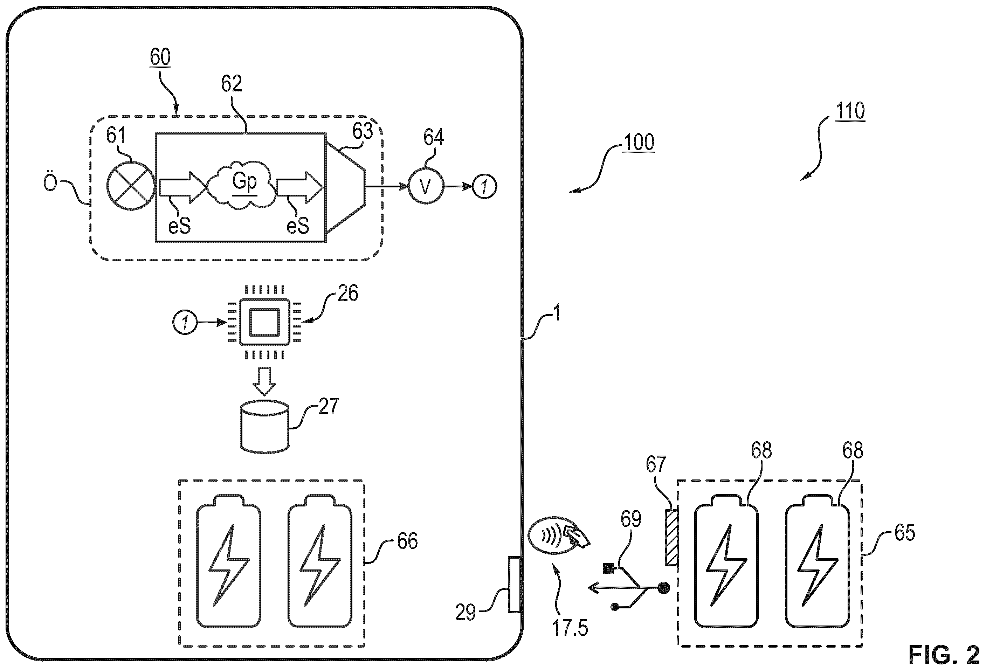

Figur 1 und Figur 2 zeigen in einer perspektivischen bzw. einer schematischen Darstellung ein solches Gasmessgerät 100. Das Gasmessgerät 100 gehört zu einer Anordnung 110, wobei die Anordnung 110 zusätzlich verschiedene Erweiterungsmodule umfasst, die nachfolgend beschrieben werden.

Ein Gehäuse 1 umschließt fluiddicht eine Messkammer 62 und nimmt in seinem Inneren mindestens einen Gassensor, bevorzugt mehrere Gassensoren, auf. Eine zu untersuchende Gasprobe Gp fließt von außen in die Messkammer und erreicht dort die Gassensoren. Bevorzugt sind die Gassensoren unterschiedlich ausgestaltet, so dass das Gasmessgerät 100 mehrere brennbare Zielgase in derselben Gasprobe Gp zu detektieren vermag. Außerdem wird optional dadurch Redundanz hergestellt, dass mindestens zwei Gassensoren dasselbe Zielgas zu detektieren vermögen, beispielsweise mit zwei unterschiedlichen Sensorprinzipien.

Der oder jeder Gassensor des Gasmessgeräts 100 misst jeweils mindestens eine Detektionsgröße. Die oder jede Detektionsgröße korreliert mit dem Vorhandensein und / oder der Konzentration jeweils eines zu detektierenden Zielgases. Die Detektionsgröße ist beispielsweise eine elektrische Spannung oder eine Stromstärke oder eine elektrische Ladung oder ein elektrischer Widerstand.

Figur 2 zeigt beispielhaft einen photo-elektrischen Sensor 60. Eine zu untersuchende Gasprobe Gp fließt aus einem zu überwachenden räumlichen Bereich durch eine Öffnung Ö hindurch in die Messkammer 62. Eine Strahlungsquelle 61 emittiert elektromagnetische Strahlung eS in die Messkammer 62 hinein. Die elektromagnetische Strahlung eS durchdringt mindestens einmal die Messkammer 62 und damit die Gasprobe Gp und trifft auf einen Detektor 63 auf. Ein zu detektierendes Zielgas in der Gasprobe Gp schwächt die elektromagnetische Strahlung eS ab. Der Detektor 63 erzeugt ein Signal, im vorliegenden Falle eine elektrische Spannung, die mit der Intensität der auftreffenden elektromagnetischen Strahlung eS korreliert. Ein Spannungssensor 64 misst die elektrische Spannung, die der Detektor 63 erzeugt. Diese elektrische Spannung ist im Beispiel von Figur 2 die Detektionsgröße, die mit der Zielgas-Konzentration korreliert.

Eine signalverarbeitende Auswerteeinheit empfängt von dem oder jedem Gassensor den jeweils gemessenen Wert der oder jeder Detektionsgröße und leitet aus dem Detektionsgrößen-Wert einen Schätzwert für die Zielgas-Konzentration her. Für diese Herleitung wendet die Auswerteeinheit einen vorgegebenen rechnerauswertbaren funktionalen Zusammenhang auf die Detektionsgröße an. Das Gasmessgerät 100 umfasst weiterhin ein ebenfalls nicht gezeigtes signalverarbeitendes Steuergerät (control unit) 26. Die Auswerteeinheit ist im Ausführungsbeispiel ein Bestandteil des Steuergeräts 26. Möglich ist auch, dass die Auswerteeinheit räumlich vom Steuergerät 26 oder sogar vom Gasmessgerät 100 entfernt ist.

Die Auswerteeinheit umfasst beispielsweise ein Programm, welches in einem Datenspeicher des Steuergeräts 26 abgespeichert ist und auf einem Prozessor des Steuergeräts 26 ablaufen kann. Wenigstens zeitweise haben das Steuergerät 26 und damit die Auswerteeinheit Lesezugriff auf einen Auswertungs-Datenspeicher 27, in dem mindestens ein funktionaler Zusammenhang zwischen der Detektionsgröße und der Zielgas-Konzentrationen abgespeichert ist.

Bevorzugt umfasst das Gasmessgerät 100 eine eigene interne Spannungsversorgungseinheit 66, welche im Inneren des Gehäuses 1 angeordnet ist und bevorzugt mehrere wiederaufladbare Batterien (Akkumulatoren) umfasst. Dadurch kann ein Benutzer das Gasmessgerät 100 mit sich führen, während er einen Bereich betritt, in dem ein Zielgas vorhanden sein kann. Das Gasmessgerät 100 lässt sich einschalten und abschalten. Möglich ist auch, das Gasmessgerät 100 stationär zu positionieren. Dank der eigenen Spannungsversorgungseinheit 66 braucht das Gasmessgerät 100 nicht mit einem stationären Spannungsversorgungsnetz verbunden zu werden. Möglich ist auch, dass das Gasmessgerät 100 ortsfest positioniert und mit einem stationären Spannungsversorgungsnetz verbunden wird. In einer Ausgestaltung lassen sich die elektrischen Verbraucher des Gasmessgeräts 100 wahlweise von einer eigenen Spannungsversorgungseinheit oder von einem stationären Spannungsversorgungsnetz versorgen.

In einer Ausgestaltung umfasst das Gasmessgerät 100 eine Steckdose 29, in die sich ein Stecker 69 eines Erweiterungsmoduls 65 einstecken lässt. Das Erweiterungsmodul 65 umfasst weiterhin eine externe Spannungsversorgungseinheit 68. Die elektrischen Verbraucher 26, 61 des Gasmessgeräts 100 werden dann von der internen Spannungsversorgungseinheit 66 mit elektrischer Energie versorgt, wenn keine externe Spannungsversorgungseinheit 68 angeschlossen ist. Wenn das Steuergerät 26 erkennt, dass ein Erweiterungsmodul 65 mit einer externen Spannungsversorgungseinheit 68 angeschlossen ist, bewirkt das Steuergerät 26, dass die elektrischen Verbraucher 26, 61 von der externen Spannungsversorgungseinheit 68 versorgt werden.

**In** einer Ausgestaltung vermag das Steuergerät 26 den elektrischen Füllstand der externen Spannungsversorgungseinheit 68 zu ermitteln und mit einer vorgegebenen unteren Füllstands-Schranke zu vergleichen. Das Steuergerät 26 bewirkt folgendes: Die elektrischen Verbraucher 26, 61 werden nur dann von der externen Spannungsversorgungseinheit 68 versorgt, wenn das Erweiterungsmodul 65 mit dem Gasmessgerät 100 verbunden ist und der gemessene Füllstand oberhalb der unteren Füllstands-Schranke liegt, und ansonsten von der internen Spannungsversorgungseinheit 66.

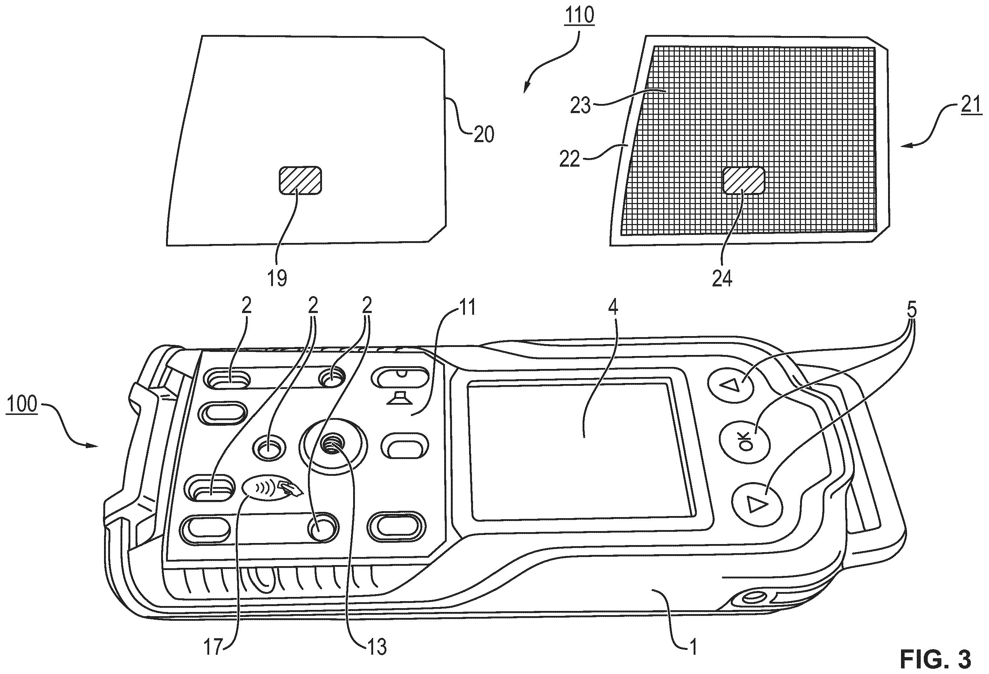

In das Gehäuse 1 ist eine Vielzahl von Einlässen 2 eingelassen, vgl. Figur 1 und Figur 3. Die Einlässe 2 stellen zusammen eine Fluidverbindung zwischen der Umgebung des Gasmessgeräts 100 und den Gassensoren im Inneren des Gehäuses 1 her, beispielsweise dem Gassensor 60 von Figur 2. In das Gehäuse 1 sind weiterhin eine Anzeigeeinheit 4 sowie mehrere Bedienelemente 5 eingelassen, vgl. Figur 1. Auf der Anzeigeeinheit 4 vermag das Gasmessgerät 100 Messergebnisse und / oder Alarme sowie Meldungen über den eigenen Zustand visuell auszugeben. In einer Ausgestaltung vermag das Gasmessgerät 100 einen Alarm zu generieren und diesen Alarm visuell und / oder akustisch und / oder haptisch (durch Vibrationen) auszugeben, falls die gemessene Konzentration eines Zielgases oder auch die Summe aller Zielgas-Konzentrationen oberhalb einer vorgegebenen Konzentrations-Schranke liegt.

In einer Anwendung diffundiert ein Fluid aus der Umgebung des Gasmessgeräts 100 durch die Einlässe 2 hindurch zu den Gassensoren im Inneren des Gehäuses 1. Diese Anwendung wird in Figur 1 und Figur 3 gezeigt. Möglich ist auch, dass eine Pumpe (nicht gezeigt) das Fluid ansaugt.

In der Regel wird das Gasmessgerät 100 zeitweise genutzt und befindet sich zeitweise in einem Ruhezustand, in dem es bevorzugt ausgeschaltet ist, sodass keine elektrische Energie verbraucht wird. Häufig wird gewünscht, dass das Gasmessgerät 100 im Ruhezustand nicht in einer Fluidverbindung mit der Umgebung steht. Daher lässt sich auf die Einlässe 2 eine Abdeckkappe 20 fluiddicht aufsetzen und wieder abnehmen. Figur 3 zeigt schematisch die Abdeckkappe 20 sowie einen annähernd rechteckigen Bereich 11 der Oberfläche des Gehäuses 1, der von der aufgesetzten Abdeckkappe 20 bedeckt ist. Bei aufgesetzter Abdeckkappe 20 lässt das Gasmessgerät 100 sich mit einer Reinigungsflüssigkeit reinigen, ohne dass die Gefahr besteht, dass Reinigungsflüssigkeit in das Innere des Gehäuses 1 gelangt. Außerdem können bei aufgesetzter Abdeckkappe 20 keine schädlichen Substanzen oder Wassertröpfchen aus der Umgebung in das Innere des Gasmessgeräts 100 gelangen. Ein Benutzer kann die Abdeckkappe 20 nach einem Einsatz auf die Einlässe 2 aufsetzen und vor einem neuen Einsatz wieder abnehmen. Selbstverständlich darf die Abdeckkappe 20 bei einem Einsatz nicht aufgesetzt sein, weil dann keine Gasprobe die Messkammer erreichen kann und das Gasmessgerät 100 ein Zielgas nicht detektieren kann.

Weiterhin lässt sich auf den Bereich 11 mit den Einlässen 2 eine ebenfalls annähernd rechteckige Gittereinheit 21 aufsetzen. Die Gittereinheit 21 reduziert das Risiko, dass ein Insekt oder ein größeres Partikel in das Innere des Gehäuses 1 gelangt und Gassensoren beschädigen könnte. Die Gittereinheit 21 umfasst ein engmaschiges Gitter 22 und einen Rahmen 23.

Möglich ist auch, auf den Bereich 11 der Einlässe 2 einen Spritzschutz (nicht gezeigt) aufzusetzen. Dieser Spritzschutz reduziert die Gefahr, dass bei einem Einsatz Flüssigkeitstropfen in das Innere des Gehäuses 1 gelangen. Bevorzugt lassen sich auf die Einlässe 2 wahlweise die Abdeckkappe 20, die Gittereinheit 21, der Spritzschutz oder keines dieser drei Bauteile aufsetzen.

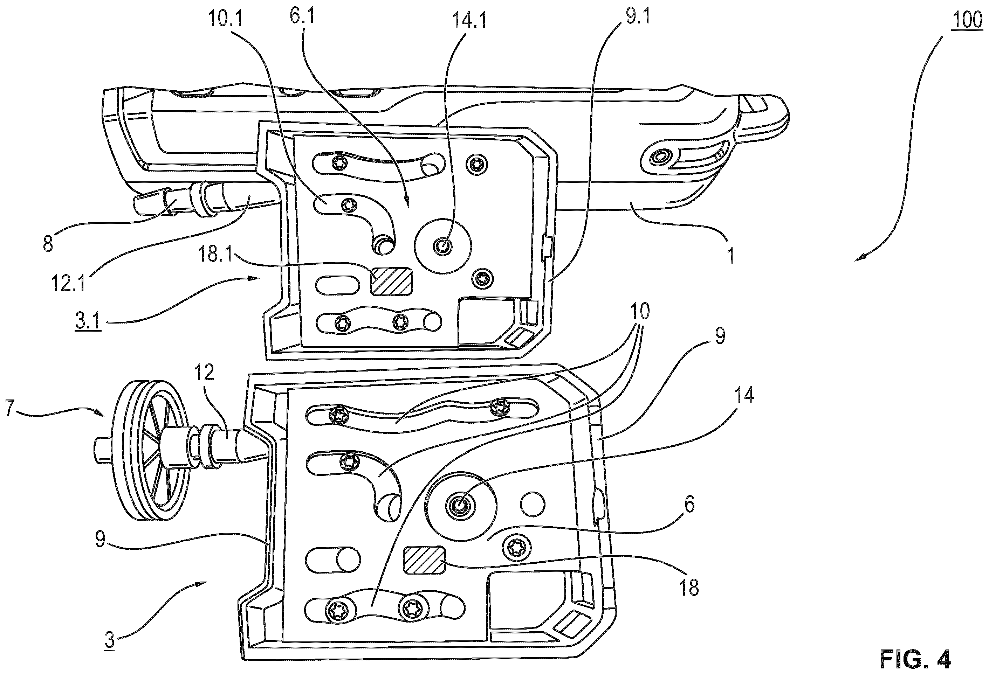

Außerdem lässt sich auf die Einlässe 2 im Gehäuse 1 ein Adapter 3 fluiddicht aufsetzen, vgl. Figur 4. Falls der Adapter 3 auf das Gehäuse 1 aufgesetzt ist, so überdeckt der Adapter 3 den Bereich 11 und unterbricht damit die Fluidverbindung zwischen den Einlässen 2 und der Umgebung des Gasmessgeräts 100. Sowohl bei aufgesetzter Abdeckkappe 20 als auch bei aufgesetztem Adapter 3 besteht keine Fluidverbindung zwischen den Gassensoren im Gehäuse 1 und der Umgebung des Gasmessgeräts 100 durch die Einlässe 2 hindurch. In Figur 3 ist ein Gewinde 13 für eine Schraube 14 zu sehen, wobei diese Schraube 14 den Adapter 3 lösbar am Gehäuse 1 zu halten vermag.

Der Adapter 3 wird während eines Einsatzes des Gasmessgeräts 100 verwendet und umfasst
- eine harte Außenschale 9 mit einem Anschlusselement in Form eines Stutzens 12,
- eine elastische Füllung 6, die von der Außenschale 9 umgeben ist, und
- eine Schraube 14.

Figur 4 zeigt den Adapter 3 (unten) und einen Teil des Gehäuses 1 (oben), wobei die elastische Füllung 6 zum Betrachter hin zeigt.

Bei aufgesetztem Adapter 3 lässt die Schraube 14 sich in das Gewinde 13 eindrehen. Die Füllung 6 befindet sich dann zwischen der Außenschale 9 und den Gassensoren. In die Füllung 6 ist eine Vielzahl von Fluidkanälen 10 eingelassen. Diese Fluidkanäle 10 stellen Fluidverbindungen zwischen dem Stutzen 12 und den Gassensoren her.

Auf den Stutzen 12 lässt sich ein Verbindungselement 7 fluiddicht aufsetzen. Dieses Verbindungselement 7 lässt sich mit einer nicht gezeigten Fluidführungseinheit, insbesondere mit einem Schlauch, fluiddicht verbinden. Mit der Fluidführungseinheit lässt sich eine Fluidfördereinheit, beispielsweise eine Pumpe, verbinden. Möglich ist auch, die Fluidfördereinheit direkt mit dem Verbindungselement 7 zu verbinden. In einer anderen Ausgestaltung ist die Fluidfördereinheit im Inneren des Gehäuses 1 angeordnet.

Dank des Schlauchs und der Fluidfördereinheit vermag das Gasmessgerät 100 eine zu untersuchende Gasprobe anzusaugen. Die Gasprobe befindet sich vor dem Ansaugen an einer Messposition, die vom Gasmessgerät 100 beabstandet ist. Das Verbindungselement 7, der Stutzen 12 und die Fluidkanäle 10 leiten die angesaugte Gasprobe zu den Sensoren im Gehäuse 1 weiter. Möglich ist, dass die Gasprobe aus einem umschlossenen Raum stammt und das Gasmessgerät 100 außerhalb dieses Raums angeordnet ist. Dadurch ist das Gasmessgerät 100 bis zu einem gewissen Grad vor mechanischen und chemischen Einwirkungen geschützt, die in dem umschlossenen Raum auftreten können. Der fluiddicht aufgesetzte Adapter 3 verhindert, dass Gas aus der Umgebung des Gasmessgeräts 100 zu den Gassensoren gelangt oder dass eine Gasprobe aus dem Inneren des Gehäuses 1 in die Umgebung gelangt.

Vor einem Einsatz wird das Gasmessgerät 100 kalibriert. Eine nicht gezeigte Kalibrierstation führt dem Gasmessgerät 100 mindestens eine Gasprobe, bevorzugt mehrere unterschiedliche Gasproben, zu, wobei die oder jede Gasprobe jeweils ein zu detektierendes Zielgas mit einer bekannten Konzentration enthält. In einer Realisierungsform umfasst die Kalibrierstation mindestens eine Druckgasflasche, wobei die oder jede Druckgasflasche jeweils ein Zielgas mit einer bekannten Konzentration enthält.

Beim Kalibrieren wird jeweils gemessen, welchen Wert eine Detektionsgröße des Gasmessgeräts 100 bei dieser Gasprobe annimmt. Aus den Messwerten und den bekannten Konzentrationen wird automatisch ein Zusammenhang zwischen der Zielgas-Konzentration und der Detektionsgröße empirisch hergeleitet und in einer rechnerauswertbaren Form im Auswertungs-Datenspeicher 27 abgespeichert. Beispielsweise wird eine Regressionsanalyse durchgeführt, oder ein neuronales Netzwerk wird trainiert.

Während der Kalibrierung ist statt des Adapters 3 ein weiterer Adapter 3.1 auf das Gehäuse 1 aufgesetzt, vgl. Figur 4. Zur Unterscheidung von dem Adapter 3, der während eines Einsatzes des Gasmessgeräts 100 verwendet wird, wird dieser weitere Adapter 3.1 "Kalibrier-Adapter" genannt. Die äußere Geometrie des Kalibrier-Adapters 3.1 stimmt mit der äußeren Geometrie des Adapters 3 überein, sodass sich auf das Gehäuse 1 wahlweise der Adapter 3 oder der Kalibrier-Adapter 3.1 aufnsetzen lässt. Auch der aufgesetzte Kalibrier-Adapter 3.1 überdeckt vollständig den Bereich 11 und unterbindet eine Fluidverbindung zur Umgebung.

Der Kalibrier-Adapter 3.1 umfasst
- eine harte Außenschale 9.1 mit einem Anschlusselement in Form eines Stutzens 12.1,
- eine elastische Füllung 6.1 und
- eine Schraube 14.1.

Figur 4 zeigt in der Mitte den Kalibrier-Adapter 3.1, wobei die Füllung 6.1 zum Betrachter hin zeigt. Die Schraube 14.1 lässt sich in das Gewinde 13 eindrehen.

In die Füllung 6.1 ist eine Vielzahl von Fluidkanälen 10.1 eingelassen. Auf den Stutzen 12.1 lässt sich ein Kalibrier-Verbindungselement 8 fluiddicht aufsetzen. Dieses Kalibrier-Verbindungselement 8 sowie optional eine nicht gezeigte Fluidführungseinheit ermöglichen es, den Kalibrier-Adapter 3.1 fluiddicht mit der Kalibrierstation zu verbinden. Eine Gasprobe fließt von der Kalibrierstation durch die optionale Fluidführungseinheit, das Kalibrier-Verbindungselement 8 und die Fluidkanäle 10.1 hindurch zu den Gassensoren.

Die Füllung 6 des Adapters 3 und die Füllung 6.1 des Kalibrier-Adapters 3.1 sind aus einem Schaumstoff hergestellt. Dieser Schaumstoff wird in fließfähiger Form in einen Hohlkörper verbracht, beispielsweise gepresst, härtet dort aus und wird dann wieder aus dem Hohlkörper entnommen. Die Füllung 6, 6.1 wird formschlüssig mit der Außenschale 9, 9.1 verbunden, beispielsweise durch Kleben, oder wird verschraubt. Optional wird aus dem Schaumstoff mindestens eine Platte hergestellt, und mehrere Exemplare der Füllung 6, 6.1 werden aus dieser Platte herausgeschnitten. Durch das Herausschneiden erhält jede Füllung 6, 6.1 die gewünschte Umfangskontur.

In einer anderen Ausgestaltung wird der Schaum auf die Außenschale 9, 9.1 aufgetragen, wobei mehrere Hohlkörper nahe der Innenseite der Außenschale 9, 9.1 gehalten werden. Dies bewirkt, dass die Fluidkanäle 10, 10.1 gebildet werden. Außerdem verbindet sich beim Aushärten der Schaum mit der Außenschale 9, 9.1.

Durch beide gerade erwähnten Fertigungsverfahren lässt sich leichter eine Füllung 6, 6.1 mit einer gewünschten Geometrie herstellen als mit einem anderen möglichen Fertigungsverfahren.

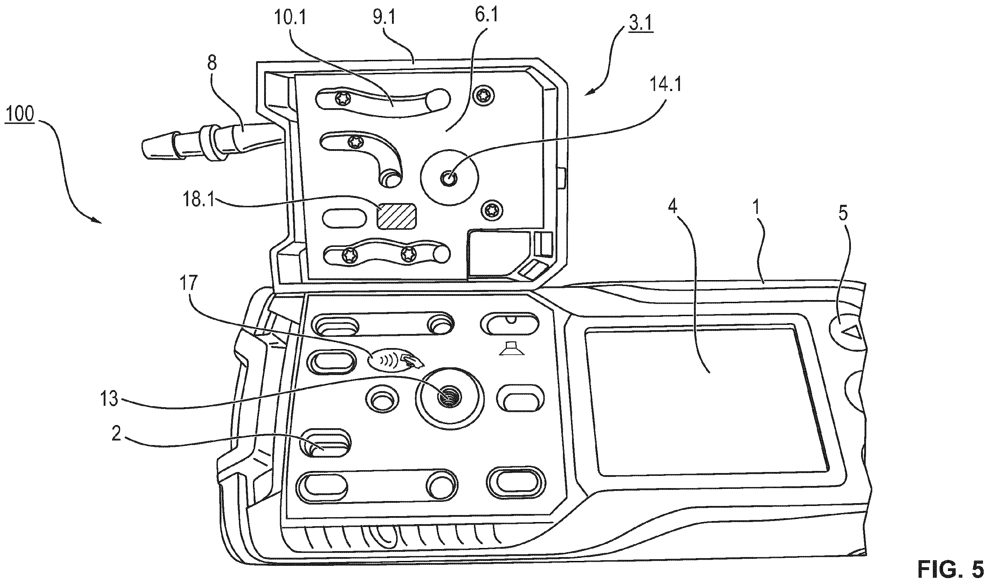

Figur 5 zeigt den Kalibrier-Adapter 3.1 (oben) und einen Teil des Gasmessgeräts 100 (unten), bevor der Kalibrier-Adapter 3.1 auf das Gehäuse 1 aufgesetzt ist. Die Füllung 6.1 zeigt zum Betrachter hin.

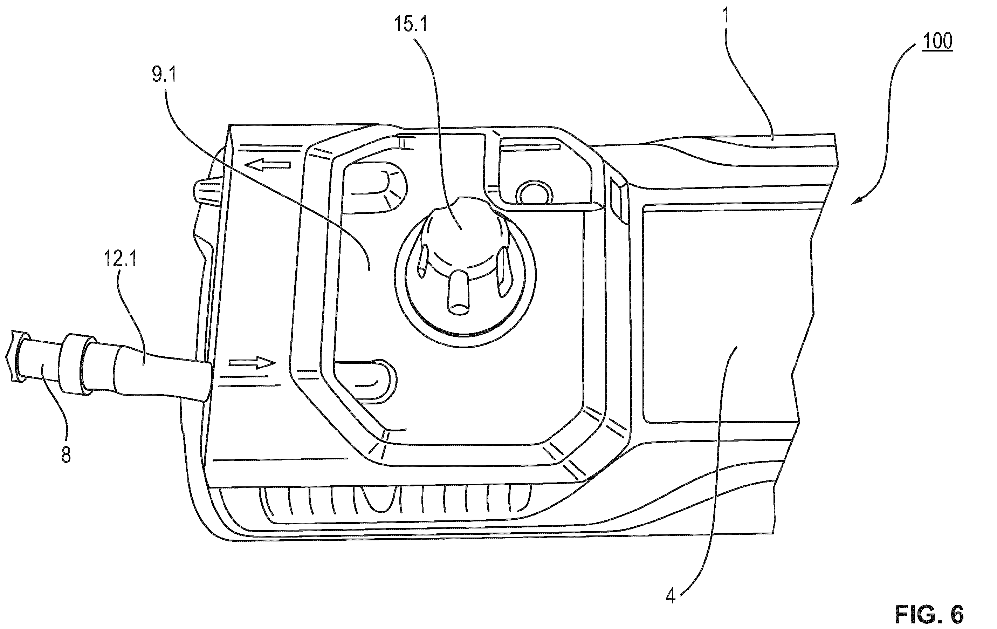

Figur 6 zeigt das Gasmessgerät 100 mit dem aufgesetzten Kalibrier-Adapter 3.1. Der Kalibrier-Adapter 3.1 verdeckt die Einlässe 2. Die Außenschale 9.1 zeigt zum Betrachter hin. Ein Knopf 15.1 ist drehbar mit der Außenschale 9.1 sowie drehfest mit der Schraube 14.1 verbunden und ermöglicht es, von außen die Schraube 14.1 zu drehen. Weiterhin sind der Stutzen 12.1 und das Kalibrier-Verbindungselement 8 zu sehen.

Mit Bezug auf die Figuren wurden unterschiedliche mögliche Konfigurationen des Gasmessgeräts 100 beschrieben. Die Arbeitsweise des Gasmessgeräts 100 hängt von der aktuellen Konfiguration ab. Nachfolgend werden einige Beispiele genannt.
- Fällt nicht unter den Anspruchsgegenstand: Bei aufgesetzter Abdeckkappe 20 vermag das Gasmessgerät 100 offensichtlich kein brennbares oder sonstiges Zielgas zu detektieren. Daher muss bei einem Einsatz des Gasmessgeräts 100 die Abdeckkappe 20 abgenommen sein. Der Vorgang, dass das Gasmessgerät 100 eingeschaltet wird, ist ein Indiz dafür, dass das Gasmessgerät 100 nunmehr dafür eingesetzt werden soll, um einen räumlichen Bereich auf mindestens ein Zielgas zu überwachen.
- Fällt nicht unter den Anspruchsgegenstand: Bei aufgesetztem Adapter 3, 3.1 benötigt eine Gasprobe Gp in der Regel mehr Zeit, um von der Messposition in das Innere des Gehäuses 1 zu gelangen, als wenn die Gasprobe Gp durch die Einlässe 2 hindurch in das Innere des Gehäuses 1 gelangt. Daher liegt ein zuverlässiges Messergebnis auch erst später vor.
- Die Auswerteeinheit wendet einen abgespeicherten funktionalen Zusammenhang, beispielsweise einen Proportionalitätsfaktor, auf den gemessenen Wert der Detektionsgröße an, um die Konzentration eines Zielgases zu ermitteln. Möglich ist, dass ein Teil eines Zielgases beim Weg durch die Fluidkanäle 10, 10.1 von der elastischen Füllung 6, 6.1 um die Fluidkanäle 10, 10.1 herum aufgesogen wird und daher die elastische Füllung 6, 6.1 einen Teil eines Zielgases in der Gasprobe Gp absorbiert. Daher hängt die Detektionsgröße nicht nur von der Zielgas-Konzentration ab, sondern auch davon, ob das Gasmessgerät 100 mit aufgesetztem Adapter 3, 3.1 oder ohne Adapter 3, 3.1 eingesetzt wird. Daher wendet die Auswerteeinheit unterschiedliche funktionale Zusammenhänge an, je nachdem ob ein Adapter 3, 3.1 aufgesetzt ist oder nicht.
- Fällt nicht unter den Anspruchsgegenstand: Auch bei aufgesetzter Gittereinheit 21 benötigt eine Gasprobe häufig mehr Zeit, um zu den Gassensoren im Gehäuse 1 zu gelangen, verglichen mit einem Zustand ohne aufgesetzte Gittereinheit 21.
- Fällt nicht unter den Anspruchsgegenstand: Der Adapter 3 ist für einen Einsatz des Gasmessgeräts 100 bestimmt, der Kalibrier-Adapter 3.1 für eine Kalibrierung. Bei einer Kalibrierung wird das Gasmessgerät 100 anders verwendet als bei einem Einsatz, nämlich in einem Kalibriermodus anstelle in einem Einsatzmodus.
- Falls das Gasmessgerät 100 mit dem Erweiterungsmodul 65 verbunden ist, werden die elektrischen Verbraucher 26, 61 von der externen Spannungsversorgungseinheit 68 versorgt, ansonsten von der internen Spannungsversorgungseinheit 66.

Das Gasmessgerät 100 lässt sich also in unterschiedlichen Konfigurationen verwenden. Diese Konfiguration lässt sich von Einsatz zu Einsatz verändern. Erfindungsgemäß vermag das Gasmessgerät 100 bis zu einem gewissen Grad automatisch seine aktuelle Konfiguration zu erkennen.

Die oben beschriebenen unterschiedlichen Konfigurationen rühren daher, dass sich ein optionales Erweiterungsmodul oder eines von mehreren möglichen Erweiterungsmodulen lösbar mit dem Gehäuse 1 des Gasmessgeräts 100 verbinden lässt. In das Gehäuse 1 ist ein Lesegerät 17, 17.5 eingelassen, das bevorzugt als eine Sende-Empfangs-Einheit ausgestaltet ist. Mindestens ein, bevorzugt jedes Erweiterungsmodul trägt einen Datenspeicher, der von außen auslesbar ist. Beispielhaft werden in Figur 2 ein Datenspeicher 67 für das Erweiterungsmodul 65 und in Figur 3 bis Figur 5 ein Datenspeicher 19 für die Abdeckkappe 20, ein Datenspeicher 24 für die Gittereinheit 21, ein Datenspeicher 18 für den Adapter 3 und ein Datenspeicher 18.1 für den Kalibrier-Adapter 3.1 gezeigt.

Das Lesegerät 17, 17.5 vermag jeden Datenspeicher 18, 18.1, 19, 24, 67 berührungslos auszulesen, vorausgesetzt der Datenspeicher 18, 18.1, 19, 24, 67 befindet sich in einem Lesebereich des Lesegeräts 17, 17.5. Um ausgelesen werden zu können, ist dieser Datenspeicher 18, 18.1, 19, 24, 67 dergestalt auf das Erweiterungsmodul 3, 3.1, 20, 21, 65 aufgebracht, dass sich bei aufgesetztem oder anderweitig verbundenem Erweiterungsmodul 3, 3.1, 20, 21, 65 der Datenspeicher 18, 18.1, 19, 24, 67 im Lesebereich des Lesegeräts 17, 17.5 befindet. Wenn andererseits das Lesegerät 17, 17.5 keinen Datenspeicher erkennt, ist das jeweilige Erweiterungsmodul auch nicht mit dem Gasmessgerät 100 verbunden.

Das Lesegerät 17, 17.5 umfasst bevorzugt
- eine Sendeeinheit,
- eine Antenne,
- eine Verbindung mit der Spannungsversorgungseinheit 66, 65 des Gasmessgeräts 100 oder mit einem stationären Spannungsversorgungsnetz und
- eine eigene Signalverarbeitungseinheit.

Im Ausführungsbeispiel ist jeder Datenspeicher 18, 18.1, 19, 24, 67 als ein Transponder-Chip ("tag"), bevorzugt als ein RFID-Chip, ausgestaltet. Das Lesegerät 17, 17.5 ist als RFID-Lesegerät ausgestaltet. Möglich ist auch, dass das Lesegerät 17, 17.5 ein anderes geeignetes Verfahren anwendet, beispielsweise Near Field Communication (NFC), Low Frequency (LF), High Frequency oder Ultra High Frequency (UHF).

In einer Realisierungsform, um Erweiterungsmodule mit Datenspeichern zu versehen, wird eine Abfolge von Datenträgern auf ein Band aufgebracht, wobei eine Oberfläche dieses Bandes mit einer Klebeschicht verbunden ist. Beispielsweise ist das Band eine Klebefolie. Jeweils ein Datenspeicher 18, 18.1, 19, 24, 67 pro Erweiterungsmodul wird auf die Klebeschicht aufgebracht. Ein Teil der Klebeschicht mit dem Datenspeicher 18, 18.1, 19, 24, 67 wird an einer bestimmten Stelle auf der Oberfläche des Erweiterungsmoduls 3, 3.1, 20, 21, 65 aufgeklebt. Bevorzugt befindet der Datenspeicher 18, 18.1, 19, 24, 67 sich zwischen dem Erweiterungsmodul 3, 3.1, 20, 21, 65 und dem Band, so dass das Band den Datenspeicher 18, 18.1, 19, 24, 67 bis zu einem gewissen Grad vor Umgebungseinflüssen schützt. In der Regel ist das Band für elektromagnetische Wellen durchlässig.

Möglich ist auch, dass ein Gehäuse oder ein sonstiger Bestandteil des Erweiterungsmoduls 3, 3.1, 20, 21, 65 oder auch das komplette Erweiterungsmodul durch Gießen hergestellt wird. Während das Bauteil (Bestandteil oder Erweiterungsmodul) hergestellt wird, wird der Datenspeicher 18, 18.1, 19, 24, 67 in das Innere dieses Bauteils verbracht und dort zeitweise gehalten. Das aushärtende Gießmaterial, aus dem das Bauteil hergestellt wird, umgibt den Datenspeicher 18, 18.1, 19, 24, 67. Der Datenspeicher 18, 18.1, 19, 24 wird also durch Umspritzen oder durch Vergießen (Potting) in das Innere des Bauteils verbracht.

Auf einem Datenspeicher 18, 18.1, 19, 24, 67 ist mindestens die Information abgespeichert, von welchem Typ das Erweiterungsmodul 3, 3.1, 20, 21, 65 ist, auf den dieser Datenspeicher 18, 18.1, 19, 24, 67 aufgebracht ist. Möglich ist, dass zusätzlich eine eindeutige Kennzeichnung abgespeichert ist, die das Erweiterungsmodul von gleichartigen Erweiterungsmodulen unterscheidet. Optional sind zusätzlich Eigenschaften des Erweiterungsmoduls 3, 3.1, 20, 21, 65 abgespeichert. Beispielsweise ist ein Proportionalitätsfaktor zwischen einer Detektionsgröße, welche das Erweiterungsmodul zu messen vermag, und einer Zielgas-Konzentration im Datenspeicher abgespeichert oder auch eine Zeitspanne, die verstreicht, bis eine Gasprobe durch das Erweiterungsmodul hindurch in das Innere des Gasmessgeräts 100 geflossen ist.

Nachfolgend wird anhand mehrerer Beispiele beschrieben, wie das Gasmessgerät 100 seine eigene aktuelle Konfiguration erkennt und während des Einsatzes automatisch nutzt. Das Steuergerät 26 des Gasmessgeräts 100 empfängt Signale von dem oder mindestens einem Lesegerät 17, 17.5 und dadurch Informationen aus einem ausgelesenen Datenspeicher 18, 18.1, 19, 24, 67, vorausgesetzt das entsprechende Erweiterungsmodul ist korrekt aufgesetzt.
- Sobald das Steuergerät 26 erkannt hat, dass das Erweiterungsmodul 65 in die Nähe des Lesegerät 17.5 gekommen ist und der Stecker 69 eingesteckt ist, bewirkt das Steuergerät 26 folgendes: Die elektrischen Verbraucher des Gasmessgeräts 100 werden von der externen Spannungsversorgungseinheit 68 versorgt. Sobald umgekehrt das Steuergerät 26 erkennt, dass das Erweiterungsmodul 65 nicht mehr mit dem Gasmessgerät 100 verbunden ist oder aber das Spannungsniveau (der elektrische Füllstand) der externen Spannungsversorgungseinheit 68 unter eine vorgegebene Schranke gefallen ist, schaltet das Steuergerät 26 wieder auf die interne Spannungsversorgungseinheit 66 um.
   Zwei Vorteile dieser Ausgestaltung sind: In der Regel lässt sich das Erweiterungsmodul 65 rascher austauschen als die interne Spannungsversorgungseinheit 66. In manchen Fällen ist es möglich, das Erweiterungsmodul 65 auch in einer explosionsgefährdeten Umgebung auszutauschen, während die interne Spannungsversorgungseinheit 66 sich nur außerhalb dieser Umgebung austauschen lässt.
- Eine in der Regel unerwünschte Situation ist aufgetreten, wenn das Gasmessgerät 100 eingeschaltet ist und das Lesegerät 17 den Datenspeicher 19 auf der Abdeckkappe 20 erkennt und ausliest. Aufgrund eines entsprechenden Signals des Lesegeräts 17 stellt das Steuergerät 26 fest, dass die Abdeckkappe 20 aufgesetzt ist. Dies ist in der Regel in Verbindung mit einem eingeschalteten Gasmessgerät 100 unerwünscht. Denn entweder will ein Benutzer das Gasmessgerät 100 zum Detektieren eines Zielgases verwenden, obwohl die Abdeckkappe 20 aufgesetzt ist. Oder der Benutzer will das Gasmessgerät 100 reinigen, obwohl es eingeschaltet ist. Beide Fälle sind unerwünscht, und daher erzeugt das Steuergerät 26 in beiden Fällen einen Alarm. Das Gasmessgerät 100 gibt diesen Alarm in einer von einem Menschen wahrnehmbaren Form aus, beispielsweise visuell auf der Anzeigeeinheit 4 sowie akustisch.
- Im Auswertungs-Datenspeicher 27 des Gasmessgeräts 100 sind verschiedene funktionale Zusammenhänge zwischen derselben Detektionsgröße und der Konzentration desselben Zielgases abgespeichert. Die Auswerteeinheit hat wenigstens zeitweise Lesezugriff auf den Auswertungs-Datenspeicher 27. Die aktuelle Konfiguration des Gasmessgeräts 100 legt fest, welchen dieser abgespeicherten funktionalen Zusammenhänge die Auswerteeinheit für welche gemessene Detektionsgröße benutzt. Die Auswerteeinheit wählt einen funktionalen Zusammenhang abhängig von einem Signal des Lesegeräts 17 aus. Falls das Gasmessgerät 100 in der aktuellen Konfiguration mehrere Gassensoren umfasst, so wendet die Auswerteeinheit mehrere abgespeicherte funktionale Zusammenhänge an.
- Ein erster funktionaler Zusammenhang wird verwendet, wenn das Lesegerät 17 keinen Datenspeicher eines Erweiterungsmoduls erkannt hat. In diesem Falle ist kein Erweiterungsmodul auf den Bereich 11 aufgesetzt, und eine Gasprobe diffundiert durch die Einlässe 2 hindurch aus der Umgebung in das Innere des Gehäuses 1.
- Ein zweiter funktionaler Zusammenhang wird verwendet, wenn das Lesegerät 17 den Datenspeicher 18 erkannt hat, was zeigt, dass der Adapter 3 auf den Bereich 11 aufgesetzt ist. Der nunmehr verwendete zweite funktionale Zusammenhang berücksichtigt einerseits, dass ein Teil eines Zielgases von der elastischen Füllung 6 absorbiert wird. Andererseits berücksichtigt die Auswerteeinheit im Steuergerät 26 automatisch, dass ein zuverlässiges Messergebnis, also ein verwendbarer Wert der Detektionsgröße, erst nach einer längeren Zeit vorliegt, weil die Gasprobe Gp durch einen Schlauch und den Adapter 3 hindurch zu den Gassensoren im Gehäuse 1 gelangen muss und nicht direkt durch die Einlässe 2 diffundieren kann, verglichen mit einem Zustand ohne aufgesetztem Adapter 3.
- In einer Ausgestaltung schaltet das Steuergerät 26 das Gasmessgerät 100 automatisch in einen Kalibriermodus um, wenn das Lesegerät 17 den Datenspeicher 18.1 erkannt hat und dadurch erkannt hat, dass der Kalibrier-Adapter 3.1 auf das Gasmessgerät 100 aufgesetzt ist. Nicht erforderlich ist, dass ein Benutzer das Gasmessgerät 100 in den Kalibriermodus umschaltet.
- Wenn das Lesegerät 17 den Datenspeicher 24 erkannt hat, steht fest, dass die Gittereinheit 21 auf den Bereich 11 aufgesetzt ist. Auch in diesem Fall berücksichtigt die Auswerteeinheit im Steuergerät 26, dass eine Gasprobe Gp länger braucht, um zu den Gassensoren zu gelangen. Das Entsprechende kann für einen aufgesetzten Spritzschutz gelten.

In der bislang beschriebenen Ausgestaltung sind die Gassensoren im Inneren des Gehäuses 1 fest angeordnet. In einer abweichenden Ausgestaltung lässt sich ein Erweiterungsmodul, welches einen externen Gassensor umfasst, lösbar mit dem Gasmessgerät - genauer gesagt: mit dem Basisteil - verbinden. Diese Ausgestaltung ermöglicht es, das gleiche Gasmessgerät mit alternativen und / oder optionalen Gassensoren zu verwenden.

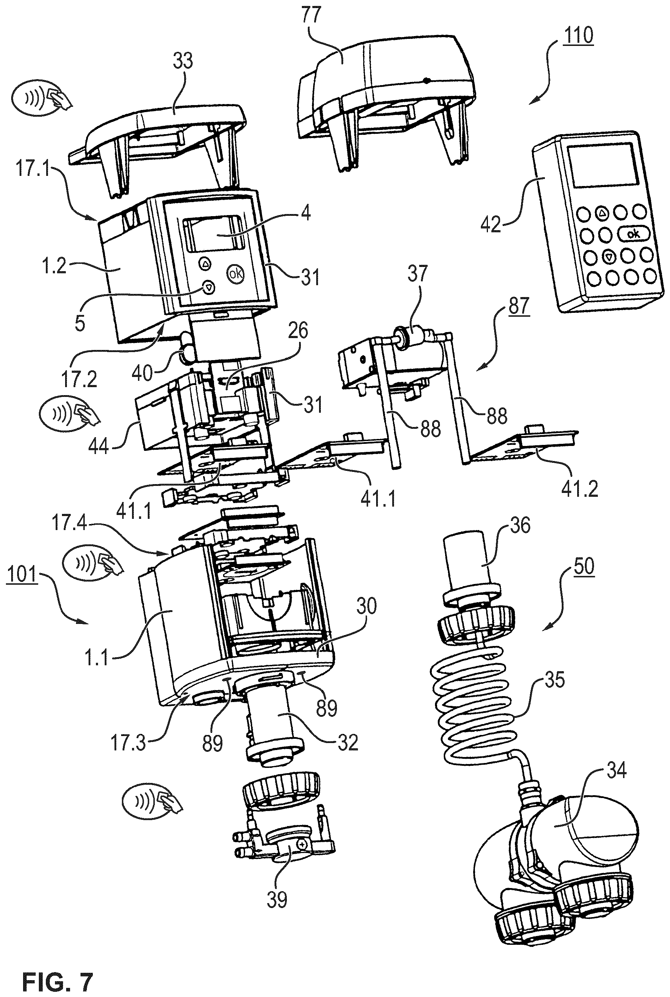

Figur 7 zeigt beispielhaft in einer Explosionsdarstellung ein solches Gasmessgerät 101. Gleiche Bezugszeichen haben die gleichen Bedeutungen wie in den vorhergehenden Figuren. Zu sehen sind:
- eine Gehäuseeinheit 30 mit einen ersten Teil 1.1 des Gehäuses 1, zwei Öffnungen 89 im ersten Teil 1.1, einem Sensoranschluss 32 und zwei Lesegeräten 17.3 und 17.4,
- eine Messeinheit 31 mit einem internen Gassensor 44, einem zweiten Teil 1.2 des Gehäuses 1, der Anzeigeeinheit 4, den Bedienelementen 5, einem abnehmbaren Deckel 33 und zwei Lesegeräten 17.1 und 17.2, wobei die Messeinheit 31 außerdem das Steuergerät 26 mit der Auswerteeinheit und weitere Elektronik (nicht gezeigt) umfasst, und
- verschiedene nachfolgend beschriebene Erweiterungsmodule.

Der interne Gassensor 44 vermag eine Detektionsgröße zu messen, welche mit der Konzentration mindestens eines Zielgases korreliert. Der interne Gassensor 44 kann so aufgebaut sein wie der Gassensor 60, der mit Bezug auf Figur 2 beschrieben wurde. Der interne Gassensor 44 kann z.B. auch ein photoakustischer oder elektrochemischer Sensor oder Wärmetönungs-Sensor sein. Verschiedene in Figur 7 gezeigte Erweiterungsmodule besitzen jeweils einen Datenspeicher, der von außen auslesbar ist. Diese Datenspeicher werden in Figur 7 nicht gezeigt.

In einer Ausgestaltung sind die Gehäuseeinheit 30 und die Messeinheit 31 fest miteinander verbunden. In einer anderen Ausgestaltung sind die beiden Einheiten 30, 31 lösbar miteinander verbunden, beispielsweise mithilfe von Schnappverschlüssen oder Rastverschlüssen oder Schraubverschlüssen. Falls die beiden Einheiten 30, 31 miteinander verbunden sind, so bilden sie ein Basisteil, mit dem sich verschiedene Erweiterungsmodule lösbar verbinden lassen. Möglich ist auch, dass nur die Gehäuseeinheit 30 ein Basisteil bildet und die Messeinheit 31 ein Erweiterungsmodul. Das Lesegerät 17.4 der Gehäuseeinheit 30 vermag bevorzugt einen Datenspeicher auf der Messeinheit 31 auszulesen.

An den Sensoranschluss 32 lässt sich ein Sensor-Erweiterungsmodul 50 mit einem externen Gassensor 34 und einem Verbindungsstück 36 lösbar anschließen und optional aufschrauben. Auch der Gassensor 34 vermag eine Detektionsgröße zu messen, die mit der Konzentration eines brennbaren Zielgases korreliert. In der gezeigten Realisierungsform lässt sich das Verbindungsstück 36 aufsetzen und einschrauben. Falls das Verbindungsstück 36 mit dem Sensoranschluss 32 verbunden ist, so wird der Gassensor 34 über eine Verbindungsleitung 35 mit elektrischer Energie versorgt.

Das Lesegerät 17.3 vermag einen Datenspeicher auf dem Sensor-Erweiterungsmodul 50 auszulesen, wobei dieser Datenspeicher sich bevorzugt am Verbindungsstück 36 befindet. Indem das Steuergerät 26 ein Signal des Lesegeräts 17.3 auswertet, erkennt das Steuergerät 26 automatisch, von welchem Typ der Gassensor 34 ist, und parametriert die Messeinheit 31 entsprechend. Möglich ist, dass der interne Gassensor 44 ein erstes Zielgas und der externe Gassensor 34 ein zweites Zielgas zu detektieren vermögen.

Auf den Sensoranschluss 32 lässt sich ein Adapter 39 für eine Pumpe (nicht gezeigt) aufstecken. Diese Pumpe vermag eine Gasprobe von einem räumlich entfernten Entnahmeort anzusaugen. Die angesaugte Gasprobe fließt beispielsweise durch einen Schlauch und den Adapter 3 von Figur 4 zu dem Gassensor 44. Das Lesegerät 17.3 vermag einen Datenspeicher auf diesem Adapter 39 auszulesen.

Falls die Messeinheit 31 von der Gehäuseeinheit 30 getrennt ist, lässt sich ein Pumpen-Erweiterungsmodul 87 mit einer Pumpe 37 von oben auf die Gehäuseeinheit 30 aufsetzen. Diese Pumpe 37 vermag eine Gasprobe anzusaugen und zu dem Gassensor 44 der Messeinheit 31 zu fördern. Bei aufgesetztem Pumpen-Erweiterungsmodul 87 führen zwei Gasleitungen 88, nämlich eine Saugleitung und eine Druckleitung, zu den beiden Öffnungen 89 an der Unterseite der Gehäuseeinheit 30. Die Förderrichtung der Pumpe 37 lässt sich bevorzugt umkehren, so dass die Gasprobe wahlweise zum internen Gassensor 44 der Messeinheit 31 oder zum externen Gassensor 34 des angeschlossenen Sensor-Erweiterungsmoduls 50 gefördert werden kann. Das Lesegerät 17.4 vermag einen Datenspeicher auf dem Pumpen-Erweiterungsmodul 87 auszulesen.

Mit der Messeinheit 31 lässt sich ein Freigabemodul 40 lösbar verbinden. Falls das Freigabemodul 40 mit der Messeinheit 31 verbunden ist, so gibt das Freigabemodul 40 zusätzliche Funktionen der Auswerteeinheit frei. Das Lesegerät 17.2 vermag einen Datenspeicher auf dem Freigabemodul 40 auszulesen und dadurch zu erkennen, ob das Freigabemodul 40 mit der Messeinheit 31 verbunden ist oder fehlt.

Außerdem lässt sich mit der Messeinheit 31 mindestens ein Kommunikationsmodul 41.1, 41.2 lösbar verbinden. Das Lesegerät 17.4 vermag einen Datenspeicher auf einem verbundenen Kommunikationsmodul 41.1, 41.2 auszulesen.

In einer Realisierungsform übersetzt das oder mindestens ein Kommunikationsmodul 41.1 mindestens ein Signal des Gasmessgeräts 101 in eine Nachricht gemäß einem vorgegebenen Kommunikationsstandard, bevorzugt gemäß einem Mobilfunkstandard. Das Kommunikationsmodul 41.1 oder eine andere Kommunikationseinheit vermögen diese Nachricht gemäß dem Kommunikationsstandard an einen räumlich entfernten Empfänger zu übermitteln. Dadurch lässt sich das Gasmessgerät 101 aus der Ferne auslesen. Beispielhaft wird als Empfänger ein tragbares Gerät 42 mit einem Display gezeigt. Mithilfe dieses Geräts 42 kann ein Benutzer aus der Ferne das Gasmessgerät 101 auslesen.

In einer anderen Realisierungsform empfängt das oder mindestens ein Kommunikationsmodul 41.2 ein Signal von einem Gassensor (nicht gezeigt), der eine Detektionsgröße zu messen vermag. Die Auswerteeinheit im Steuergerät 26 aktiviert einen entsprechenden funktionalen Zusammenhang zwischen dieser Detektionsgröße und einer Zielgas-Konzentration, falls ein solches Kommunikationsmodul detektiert ist. Dieser Gassensor lässt sich räumlich entfernt von dem Gasmessgerät 101 anordnen. Die Messeinheit 31 des Gasmessgeräts 101 vermag ein Signal von dem räumlich entfernten Gassensor auszuwerten. Diese beiden Realisierungsformen lassen sich miteinander kombinieren.

Bevorzugt ist an der Unterseite des Deckels 33 ein Datenspeicher angeordnet. Das Lesegerät 17.1 vermag diesen Datenspeicher am Deckel 33 auszulesen. Falls das Lesegerät 17.1 keinen Datenspeicher detektiert, so wird bevorzugt ein Alarm generiert und ausgegeben. Denn das Fehlen eines Deckels 33 ist eine unerwünschte Situation.

Der Deckel 33 lässt sich durch ein Erweiterungsmodul 77 ersetzen. Das Erweiterungsmodul 77 nimmt die mechanische Funktion des Deckels 33 wahr und enthält außerdem ein optionales Gerät, beispielsweise einen Temperatursensor oder einen Feuchtesensor oder einen Drucksensor oder einen Sensor für eine sonstige Umgebungsbedingung. Das Lesegerät 17.1 vermag einen Datenspeicher auf der Unterseite des Erweiterungsmoduls 77 auszulesen. Die Auswerteeinheit im Steuergerät 26 aktiviert einen anderen funktionalen Zusammenhang in dem oben erwähnten Datenspeicher für funktionale Zusammenhänge. Denn wenn die Umgebungstemperatur oder die Umgebungsfeuchte oder der Umgebungsdruck oder die sonstige Umgebungsbedingung gemessen werden, so lässt sich ein funktionaler Zusammenhang anwenden, in dem die Temperatur bzw. die Feuchte als eine gemessene Größe auftritt. Falls ein solches Erweiterungsmodul 77 nicht detektiert wird, so wird ein vorgegebener oder von einem Benutzer eingestellter Standardwert für die Umgebungsbedingung verwendet.

Bevorzugt ermittelt das Steuergerät 26 die aktuelle Konfiguration des Gasmessgeräts 101, ermittelt also, welche Erweiterungsmodule gemäß den Signalen der Lesegeräte 17, 17.1, ..., 17.4 aktuell mit dem eigentlichen Gasmessgerät 101 verbunden sind. Ein Wartungstechniker kann die aktuelle Konfiguration des Gasmessgeräts 101 auslesen. Bevorzugt autorisiert sich der Wartungstechniker mit Hilfe eines Datenspeichers auf einer persönlichen Karte, indem er diese persönliche Karte an ein Lesegerät 17, 17.1, ..., 17.4 hält.

### Bezugszeichenliste

| | |
|---|---|
| 1 | Gehäuse des Gasmessgeräts 100, nimmt die Anzeigeeinheit 4 und die Bedienelemente 5 auf, umgibt den Gassensor 60, hat die Einlässe 2 und das Gewinde 13 |
| 2 | Einlässe im Gehäuse 1, stellen jeweils eine Fluidverbindung zwischen einem Sensor des Gasmessgeräts 100 und der Umgebung her, sind im Bereich 11 des Gehäuses 1 angeordnet |
| 3 | Adapter, umfasst die Außenschale 9, die Füllung 6 und den Stutzen 12, lässt sich lösbar auf das Gehäuse 1 aufsetzen und überdeckt dann den Bereich 11, wird bei einem Einsatz des Gasmessgeräts 100 verwendet |
| 3.1 | Kalibrier-Adapter, umfasst die Außenschale 9.1, die Füllung 6.1 und den Stutzen 12.1, lässt sich lösbar auf das Gehäuse 1 aufsetzen und überdeckt dann den Bereich 11, wird bei einer Kalibrierung des Gasmessgeräts 100 verwendet |
| 4 | Anzeigeeinheit im Gehäuse 1 und in der Messeinheit 31, zeigt Messergebnisse an |
| 5 | Bedienelemente im Gehäuse 1 und in der Messeinheit 31 |
| 6 | Füllung des Adapters 3, ist aus einem Schaumstoff hergestellt |
| 6.1 | Füllung des Kalibrier-Adapters 3.1, ist aus einem Schaumstoff hergestellt |
| 7 | Verbindungselement, das sich auf den Stutzen 12 aufsetzen und mit einem Schlauch verbinden lässt |
| 8 | Verbindungselement, das sich auf den Stutzen 12.1 aufsetzen und mit einer Kalibrierstation verbinden lässt |
| 9 | Außenschale des Adapters 3 |
| 9.1 | Außenschale des Adapters 3.1 |
| 10 | Fluidkanäle in der Füllung 6 |
| 10.1 | Fluidkanäle in der Füllung 6.1 |
| 11 | Bereich im Gehäuse 1 über den Einlässen 2, den der aufgesetzte Adapter 3, 3.1 und die aufgesetzte Abdeckkappe 20 abdecken |
| 12 | Stutzen an der Außenschale 9 |
| 12.1 | Stutzen an der Außenschale 9.1 |
| 13 | Gewinde im Gehäuse 1, in das sich die Schraube 14, 14.1 einschrauben lässt |
| 14 | Schraube des Adapters 3, lässt sich in das Gewinde 13 einschrauben |
| 14.1 | Schraube des Kalibrier-Adapters 3.1, lässt sich in das Gewinde 13 einschrauben |
| 15.1 | Knopf für die Schraube 14.1 |
| 17 | RFID-Lesegerät im Gehäuse 1 |
| 17.1, 17.2 | RFID-Lesegeräte an der Messeinheit 31 |
| 17.3, 17.4 | RFID-Lesegeräte an der Gehäuseeinheit 30 |
| 17.5 | RFID-Lesegerät im Gehäuse 1, erkennt das Erweiterungsmodul 65 mit der externen Spannungsversorgungseinheit 68 |
| 18 | RFID-Chip auf dem Adapter 3 |
| 18.1 | RFID-Chip auf dem Kalibrier-Adapter 3.1 |
| 19 | RFID-Chip auf der Abdeckkappe 20 |
| 20 | Abdeckkappe für die Einlässe 2, überdeckt dann den Bereich 11, umfasst den RFID-Chip 19 |
| 21 | Gittereinheit, umfasst das Gitter 22, den Rahmen 23 und den RFID-Chip 24, lässt sich auf die Einlässe 2 aufsetzen, überdeckt dann den Bereich 11 |
| 22 | engmaschiges Gitter der Gittereinheit 21 |
| 23 | Rahmen für das Gitter 22 |
| 24 | RFID-Chip auf der Gittereinheit 21 |
| 26 | signalverarbeitendes Steuergerät mit einer Auswerteeinheit, wobei das Steuergerät die aktuelle Konfiguration des Gasmessgeräts 100, 101 erkennt und die Auswerteeinheit eine Zielgas-Konzentration ermittelt |
| 27 | Auswertungs-Datenspeicher, auf den die Auswerteeinheit Lesezugriff hat |
| 29 | Steckdose im Gehäuse 1, vermag den Stecker 69 aufzunehmen |
| 30 | Gehäuseeinheit, umfasst den Teil 1.1 des Gehäuses 1, den Sensoranschluss 32, die Öffnungen 89 und die RFID-Lesegeräte 17.3 und 17.4 |
| 31 | Messeinheit, umfasst den Teil 1.2 des Gehäuses 1, den Gassensor 44 und die RFID-Lesegeräte 17.1 und 17.2 |
| 32 | Sensoranschluss des Gehäuseteils 30 |
| 33 | Deckel, lässt sich von oben auf die Messeinheit 31 aufsetzen |
| 34 | Gassensor, gehört zum Sensor-Erweiterungsmodul 50 |
| 35 | elektrische Verbindungsleitung für den Gassensor 34, gehört zum Sensor-Erweiterungsmodul 50 |
| 36 | Verbindungsstück des Sensor-Erweiterungsmoduls 50, lässt sich auf den Sensoranschluss 32 aufsetzen |
| 37 | Pumpe, gehört zum Pumpen-Erweiterungsmodul 87 |
| 39 | Adapter für eine nicht gezeigte Pumpe, lässt sich auf den Sensoranschluss 32 aufsetzen |
| 40 | Freigabemodul, gibt Funktionen der Auswerteeinheit frei |
| 41.1 | Kommunikationsmodul, vermag Signale von Gassensoren des Gasmessgeräts 101 in eine Nachricht gemäß einem Kommunikationsstandard zu übersetzen und zu veranlassen, dass die Nachricht an einen räumlich entfernten Empfänger übermittelt wird |
| 41.2 | Kommunikationsmodul, vermag ein Signal von einem räumlich entfernten Gassensor zu empfangen und an die Messeinheit 31 weiterzuleiten |
| 42 | räumlich entferntes Gerät, vermag Signale von Gassensoren des Gasmessgeräts 101 zu empfangen und in mindestens einer von einem Menschen wahrnehmbaren Form auszugeben |
| 44 | Gassensor der Messeinheit 31 |
| 50 | Sensor-Erweiterungsmodul, umfasst den Gassensor 34, die Verbindungsleitung 35 und das Verbindungsstück 36, lässt sich lösbar mit dem Sensoranschluss 32 verbinden |
| 60 | photo-elektrischer Gassensor des Gasmessgeräts 100 |
| 61 | Strahlungsquelle des Gassensors 60 |
| 62 | Messkammer des Gassensors 60 |
| 63 | Detektor des Gassensors 60 |
| 64 | Spannungssensor des Gassensors 60, misst die elektrische Spannung des Detektors 63 als die Detektionsgröße |
| 65 | Erweiterungsmodul mit der externen Spannungsversorgungseinheit 68, dem Stecker 69 und dem Datenspeicher 67 |
| 66 | interne Spannungsversorgungseinheit des Gasmessgeräts 100 |
| 67 | Datenspeicher des Erweiterungsmoduls 65 |
| 68 | Spannungsversorgungseinheit des Erweiterungsmoduls 65 |
| 69 | Stecker des Erweiterungsmoduls 65 |
| 77 | Erweiterungsmodul, welches den Deckel 33 ersetzen kann, umfasst in einer Ausgestaltung einen Sensor für eine Umgebungsbedingung |
| 87 | Pumpen-Erweiterungsmodul, umfasst die Pumpe 37 und die Leitungen 88, lässt sich von oben auf die Messeinheit 31 aufsetzen |
| 88 | Leitungen des Pumpen-Erweiterungsmoduls 87, lassen sich durch die Gehäuseeinheit 30 durchführen und ragen dann aus den Öffnungen 89 heraus |
| 89 | Öffnungen in der Gehäuseeinheit 30, durch die hindurch sich die Leitungen 88 führen lassen |
| 100 | Gasmessgerät, umfasst das Gehäuse 1, den Adapter 3, 3.1, die Anzeigeeinheit 4, die Bedienelemente 5 und das RFID-Lesegerät 17 sowie nicht gezeigte Gassensoren |
| 101 | Gasmessgerät, umfasst die Gehäuseeinheit 30, die Messeinheit 31 und die RFID-Lesegeräte 17.1, ..., 17.4 |
| 110 | Anordnung, umfasst ein Gasmessgerät 100, 101 sowie verschiedene Erweiterungsmodule 3, 3.1, 20, 21, 36, 40, 41.1, 41.2, 50, 77 |
| eS | elektromagnetische Strahlung, von der Strahlungsquelle 61 emittiert, durchdringt die Messkammer 62 und die Gasprobe Gp, trifft auf den Detektor 63 auf |
| Gp | Gasprobe in der Messkammer 62 |
| Ö | Öffnung in der Messkammer 62, durch die hindurch die Gasprobe Gp in die Messkammer 62 gelangt |

## Patentansprüche

1. Anordnung (110) umfassend
- ein Gasmessgerät (100, 101) und
- mindestens ein Erweiterungsmodul (3, 3.1, 20, 21, 36, 40, 41.1, 41.2, 50, 65, 77),
wobei das Gasmessgerät (100, 101)
- ein Basisteil (1, 30, 31),
- mindestens einen Gassensor (34, 44),
- eine signalverarbeitende Auswerteeinheit (26) und
- mindestens ein Lesegerät (17, 17.1, ..., 17.4, 17.5)
umfasst,
wobei der oder jeder Gassensor (34, 44) dazu ausgestaltet ist, jeweils mindestens eine Detektionsgröße zu messen, welche mit der Konzentration mindestens eines Zielgases korreliert,
wobei die Auswerteeinheit (26) dazu ausgestaltet ist,
- die jeweilige Konzentration mindestens eines Zielgases oder die summierte Konzentration von mehreren Zielgasen zu ermitteln und
- für die oder jede Ermittlung einer Zielgas-Konzentration mindestens einen gemessenen Wert der oder einer Detektionsgröße zu verwenden,
wobei das oder jedes Erweiterungsmodul (3, 3.1, 20, 21, 36, 40, 41.1, 41.2, 50, 65, 77) sich lösbar mit dem Basisteil (1, 30, 31) verbinden lässt,
wobei das oder mindestens ein Erweiterungsmodul (3, 3.1, 20, 21, 36, 40, 41.1, 41.2, 50, 65, 77) einen Erweiterungsmodul-Datenspeicher (18, 18.1, 19, 24, 67) umfasst, in welchem eine Kennzeichnung des Erweiterungsmoduls (3, 3.1, 20, 21, 36, 40, 41.1, 41.2, 50, 65, 77) abgespeichert ist,
wobei das oder jedes Lesegerät (17, 17.1, ..., 17.4, 17.5) dazu ausgestaltet ist, dann, wenn das oder ein Erweiterungsmodul (3, 3.1, 20, 21, 36, 40, 41.1, 41.2, 50, 65, 77) mit dem Basisteil (1, 30, 31) verbunden ist, berührungslos den Erweiterungsmodul-Datenspeicher (18, 18.1, 19, 24, 67) dieses Erweiterungsmoduls (3, 3.1, 20, 21, 36, 40, 41.1, 41.2, 50, 65, 77) auszulesen,
wobei das Gasmessgerät (100, 101) dazu ausgestaltet ist, abhängig von einem Signal des oder eines Lesegeräts festzustellen, ob das oder ein Erweiterungsmodul (3, 3.1, 20, 21, 36, 40, 41.1, 41.2, 50, 65, 77), welches einen Erweiterungsmodul-Datenspeicher (18, 18.1, 19, 24, 67) umfasst, mit dem Basisteil (1, 30, 31) verbunden ist oder nicht,
wobei das Gasmessgerät (100, 101) weiterhin dazu ausgestaltet ist, dann, wenn ein solches Erweiterungsmodul (3, 3.1, 20, 21, 36, 40, 41.1, 41.2, 50, 65, 77) mit dem Basisteil (1, 30, 31) verbunden ist,
- die abgespeicherte Kennzeichnung zu ermitteln und
- abhängig von dem Vorhandensein und / oder der Art der im Erweiterungsmodul-Datenspeicher (18, 18.1, 19, 24, 67) abgespeicherten und ermittelten Kennzeichnung einen Vorgang auszulösen,
**dadurch gekennzeichnet, dass**
das oder mindestens ein Erweiterungsmodul (3) eine Abhängigkeit der Detektionsgröße von der Zielgas-Konzentration verändert und / oder die Ermittlung der Zielgas-Konzentration abhängig von der Detektionsgröße beeinflusst,
wobei das Gasmessgerät (100, 101) einen Auswertungs-Datenspeicher (27) umfasst,
in dem ein erster und mindestens ein weiterer funktionaler Zusammenhang zwischen der Detektionsgröße und der Zielgas-Konzentration abgespeichert sind,
wobei die Auswerteeinheit (26) wenigstens zeitweise Lesezugriff auf den Auswertungs-Datenspeicher (27) aufweist und
wobei die Auswerteeinheit (26) dazu ausgestaltet ist,
- dann, wenn dieses Erweiterungsmodul (3) mit dem Basisteil (1, 30, 31) verbunden ist, den ersten funktionalen Zusammenhang auf einen gemessenen Wert der oder einer Detektionsgröße anzuwenden, und
- dann, wenn dieses Erweiterungsmodul (3) nicht mit dem Basisteil (1, 30, 31) verbunden ist, den oder einen weiteren funktionalen Zusammenhang auf den gemessenen Wert dieser Detektionsgröße anzuwenden.

2. Anordnung (110) nach Anspruch 1,
**dadurch gekennzeichnet, dass**
das oder ein Erweiterungsmodul einen Sensor für eine Umgebungsbedingung umfasst und
im ersten funktionalen Zusammenhang diese Umgebungsbedingung auftritt, während im weiteren funktionalen Zusammenhang ein Standardwert für diese Umgebungsbedingung auftritt.

3. Anordnung (110) nach einem der vorhergehenden Ansprüche,
**dadurch gekennzeichnet, dass**
das oder mindestens ein Erweiterungsmodul (50) einen weiteren Gassensor (34) umfasst,
wobei der weitere Gassensor (34), der ein Bestandteil dieses Erweiterungsmoduls (50) ist, dazu ausgestaltet ist, eine Detektionsgröße zu messen, welche mit der Konzentration eines vorgegebenen Zielgases korreliert.

4. Anordnung (110) nach einem der vorhergehenden Ansprüche,
**dadurch gekennzeichnet, dass**
das oder mindestens ein Erweiterungsmodul (65) eine externe Spannungsversorgungseinheit (68) umfasst,
wobei das Gasmessgerät (100, 101) dazu ausgestaltet ist, dann, wenn dieses Erweiterungsmodul (65) mit dem Basisteil (1, 30, 31) verbunden ist,
mindestens einen, bevorzugt jeden, elektrischen Verbraucher (61, 26) des Gasmessgeräts (100, 101) aus der externen Spannungsversorgungseinheit (68) zu versorgen.

5. Anordnung (110) nach Anspruch 4,
**dadurch gekennzeichnet, dass**
das Gasmessgerät (100, 101) eine interne Spannungsversorgungseinheit (66) umfasst,
wobei das Gasmessgerät (100, 101) dazu ausgestaltet ist,
- dann, wenn dieses Erweiterungsmodul (65) mit dem Basisteil (1, 30, 31) verbunden ist, den oder mindestens einen elektrischen Verbraucher (61, 26) aus der externen Spannungsversorgungseinheit (68) zu versorgen und
- ansonsten aus der internen Spannungsversorgungseinheit (66).

6. Konfigurierungs-Verfahren zum Konfigurieren eines Gasmessgeräts (100, 101),
wobei das Gasmessgerät (100, 101)
- ein Basisteil (1, 30, 31),
- mindestens einen Gassensor (34, 44),
- eine signalverarbeitende Auswerteeinheit (26) und
- mindestens ein Lesegerät (17, 17.1, ..., 17.4, 17.5)
umfasst,
wobei der oder jeder Gassensor (34, 44) dazu ausgestaltet ist, jeweils mindestens eine Detektionsgröße zu messen, welche mit der Konzentration mindestens eines Zielgases korreliert,
wobei die Auswerteeinheit (26) dazu ausgestaltet ist,
- die Konzentration mindestens eines Zielgases oder die summierte Konzentration von mehreren Zielgasen zu ermitteln und
- für die oder jede Ermittlung einer Zielgas-Konzentration mindestens einen gemessenen Wert der oder einer Detektionsgröße zu verwenden,
wobei mindestens ein Erweiterungsmodul (3, 3.1, 20, 21, 36, 40, 41.1, 41.2, 50, 65, 77) bereitgestellt wird,
wobei das oder jedes bereitgestellte Erweiterungsmodul (3, 3.1, 20, 21, 36, 40, 41.1, 41.2, 50, 65, 77)
- sich lösbar mit dem Basisteil (1, 30, 31) verbinden lässt und
- einen Erweiterungsmodul-Datenspeicher (18, 18.1, 19, 24, 67) umfasst, in welchem eine Kennzeichnung des Erweiterungsmoduls (3, 3.1, 20, 21, 36, 40, 41.1, 41.2, 50, 65, 77) abgespeichert ist,
wobei das Konfigurierungs-Verfahren die Schritte umfasst, dass
ein Erweiterungsmodul (3, 3.1, 20, 21, 36, 40, 41.1, 41.2, 50, 65, 77) mit einem Erweiterungsmodul-Datenspeicher (18, 18.1, 19, 24, 67) lösbar mit dem Basisteil (1, 30, 31) verbunden wird,
das oder ein Lesegerät (17, 17.1, ..., 17.4, 17.5) berührungslos den Erweiterungsmodul-Datenspeicher (18, 18.1, 19, 24, 67) dieses Erweiterungsmoduls (3, 3.1, 20, 21, 36, 40, 41.1, 41.2, 50, 65, 77) ausliest und
das Gasmessgerät (100, 101)
- abhängig von einem Signal des Lesegeräts (17, 17.1, ..., 17.4, 17.5) feststellt, dass das Erweiterungsmodul mit dem Basisteil (1, 30, 31) verbunden ist,
- die im Erweiterungsmodul-Datenspeicher (18, 18.1, 19, 24, 67) abgespeicherte Kennzeichnung ermittelt und
- abhängig von dem Vorhandensein und / oder der Art der im Erweiterungsmodul-Datenspeicher (18, 18.1, 19, 24, 67) abgespeicherten und ermittelten Kennzeichnung einen Vorgang auslöst,
**dadurch gekennzeichnet, dass**
das oder mindestens ein Erweiterungsmodul (3) eine Abhängigkeit der Detektionsgröße von der Zielgas-Konzentration verändert und / oder die Ermittlung der Zielgas-Konzentration abhängig von der Detektionsgröße beeinflusst,
wobei das Gasmessgerät (100, 101) einen Auswertungs-Datenspeicher (27) umfasst,
in dem ein erster und mindestens ein weiterer funktionaler Zusammenhang zwischen der Detektionsgröße und der Zielgas-Konzentration abgespeichert sind,
wobei die Auswerteeinheit (26) wenigstens zeitweise Lesezugriff auf den Auswertungs-Datenspeicher (27) aufweist und
wobei der Vorgang den Schritt umfasst, dass die Auswerteeinheit (26)
- dann, wenn dieses Erweiterungsmodul (3) mit dem Basisteil (1, 30, 31) verbunden ist, den ersten funktionalen Zusammenhang auf einen gemessenen Wert der oder einer Detektionsgröße anwendet, und
- dann, wenn dieses Erweiterungsmodul (3) nicht mit dem Basisteil (1, 30, 31) verbunden ist, den oder einen weiteren funktionalen Zusammenhang auf den gemessenen Wert dieser Detektionsgröße anwendet.

## Claims

1. Arrangement (110) comprising
- a gas measuring device (100, 101) and
- at least one extension module (3, 3.1, 20, 21, 36, 40, 41.1, 41.2, 50, 65, 77), wherein the gas measuring device (100, 101) comprises
- a main part (1, 30, 31),
- at least one gas sensor (34, 44),
- a signal processing evaluation unit (26) and
- at least one reading device (17, 17.1, ..., 17.4, 17.5),
wherein the or each gas sensor (34, 44) is configured to measure in each case at least one detection variable which correlates with the concentration of at least one target gas,
wherein the evaluation unit (26) is configured
- to ascertain the relevant concentration of at least one target gas or the total concentration of a plurality of target gases and
- to use at least one measured value of the or a detection variable for the or each ascertainment of a target gas concentration,
wherein the or each extension module (3, 3.1, 20, 21, 36, 40, 41.1, 41.2, 50, 65, 77) is detachably connectible to the main part (1, 30, 31),
wherein the or at least one extension module (3, 3.1, 20, 21, 36, 40, 41.1, 41.2, 50, 65, 77) comprises an extension module data memory (18, 18.1, 19, 24, 67) in which an identifier of the extension module (3, 3.1, 20, 21, 36, 40, 41.1, 41.2, 50, 65, 77) is stored,
wherein the or each reading device (17, 17.1, ..., 17.4, 17.5) is configured to read out the extension module data memory (18, 18.1, 19, 24, 67) of this extension module (3, 3.1, 20, 21, 36, 40, 41.1, 41.2, 50, 65, 77) in a contactless manner, if the or an extension module (3, 3.1, 20, 21, 36, 40, 41.1, 41.2, 50, 65, 77) is connected to the main part (1, 30, 31),
wherein the gas measuring device (100, 101) is configured to establish, depending on a signal from the or a reading device, whether or not the or an extension module (3, 3.1, 20, 21, 36, 40, 41.1, 41.2, 50, 65, 77) which comprises an extension module data memory (18, 18.1, 19, 24, 67) is connected to the main part (1, 30, 31),
wherein the gas measuring device (100, 101), if such an extension module (3, 3.1, 20, 21, 36, 40, 41.1, 41.2, 50, 65, 77) is connected to the main part (1, 30, 31), is further configured
- to ascertain the stored identifier and
- to trigger a process depending on the presence and/or on the type of the identifier stored and ascertained in the extension module data memory (18, 18.1, 19, 24, 67),
**characterized in that**
the or at least one extension module (3) changes a dependence of the detection variable on the target gas concentration and/or influences the ascertainment of the target gas concentration depending on the detection variable,
wherein the gas measuring device (100, 101) comprises an evaluation data memory (27),
in which a first and at least one further functional relationship between the detection variable and the target gas concentration are stored,
wherein the evaluation unit (26) has at least temporary read access to the evaluation data memory (27) and
wherein the evaluation unit (26) is configured
- to apply the first functional relationship to a measured value of the or of a detection variable if this extension module (3) is connected to the main part (1, 30, 31), and
- to apply the or a further functional relationship to the measured value of this detection variable if this extension module (3) is not connected to the main part (1, 30, 31).

2. Arrangement (110) according to claim 1,
**characterized in that**
the or an extension module comprises a sensor for an ambient condition and
this ambient condition occurs in the first functional relationship, while a default value for this ambient condition occurs in the further functional relationship.

3. Arrangement (110) according to either of the preceding claims,
**characterized in that**
the or at least one extension module (50) comprises a further gas sensor (34),
wherein the further gas sensor (34), which is a component of this extension module (50), is configured to measure a detection variable which correlates with the concentration of a specified target gas.

4. Arrangement (110) according to any of the preceding claims,
**characterized in that**
the or at least one extension module (65) comprises an external power supply unit (68),
wherein, if this extension module (65) is connected to the main part (1, 30, 31), the gas measuring device (100, 101) is configured
to supply at least one, preferably each, electrical load (61, 26) of the gas measuring device (100, 101) from the external power supply unit (68).

5. Arrangement (110) according to claim 4,
**characterized in that**
the gas measuring device (100, 101) comprises an internal power supply unit (66),
wherein the gas measuring device (100, 101) is configured,
- if this extension module (65) is connected to the main part (1, 30, 31), to supply the or at least one electrical load (61, 26) from the external power supply unit (68) and
- otherwise from the internal power supply unit (66).

6. Configuration method for configuring a gas measuring device (100, 101),
wherein the gas measuring device (100, 101) comprises
- a main part (1, 30, 31),
- at least one gas sensor (34, 44),
- a signal processing evaluation unit (26) and
- at least one reading device (17, 17.1, ..., 17.4, 17.5),
wherein the or each gas sensor (34, 44) is configured to measure in each case at least one detection variable which correlates with the concentration of at least one target gas,
wherein the evaluation unit (26) is configured
- to ascertain the concentration of at least one target gas or the total concentration of a plurality of target gases and
- to use at least one measured value of the or a detection variable for the or each ascertainment of a target gas concentration,
wherein at least one extension module (3, 3.1, 20, 21, 36, 40, 41.1, 41.2, 50, 65, 77) is provided,
wherein the or each provided extension module (3, 3.1, 20, 21, 36, 40, 41.1, 41.2, 50, 65, 77)
- is detachably connectible to the main part (1, 30, 31) and
- comprises an extension module data memory (18, 18.1, 19, 24, 67) in which an identifier of the extension module (3, 3.1, 20, 21, 36, 40, 41.1, 41.2, 50, 65, 77) is stored,
wherein the configuration method comprises the steps of
detachably connecting an extension module (3, 3.1, 20, 21, 36, 40, 41.1, 41.2, 50, 65, 77) having an extension module data memory (18, 18.1, 19, 24, 67) to the main part (1, 30, 31),
the or a reading device (17, 17.1, ..., 17.4, 17.5) reading out the extension module data memory (18, 18.1, 19, 24, 67) of this extension module (3, 3.1, 20, 21, 36, 40, 41.1, 41.2, 50, 65, 77) in a contactless manner and
the gas measuring device (100, 101)
- establishing, depending on a signal from the reading device (17, 17.1, ..., 17.4, 17.5), that the extension module is connected to the main unit (1, 30, 31),
- ascertaining the identifier stored in the extension module data memory (18, 18.1, 19, 24, 67) and
- triggering a process depending on the presence and/or type of the identifier stored and ascertained in the extension module data memory (18, 18.1, 19, 24, 67),
**characterized in that**
the or at least one extension module (3) changes a dependence of the detection variable on the target gas concentration and/or influences the ascertainment of the target gas concentration depending on the detection variable,
wherein the gas measuring device (100, 101) comprises an evaluation data memory (27),
in which a first and at least one further functional relationship between the detection variable and the target gas concentration are stored,
wherein the evaluation unit (26) has at least temporary read access to the evaluation data memory (27) and
wherein the process comprises the step of the evaluation unit (26)
- applying the first functional relationship to a measured value of the or of a detection variable if this extension module (3) is connected to the main part (1, 30, 31), and
- applying the or a further functional relationship to the measured value of this detection variable if this extension module (3) is not connected to the main part (1, 30, 31).

## Revendications

1. Agencement (110) comprenant
- un appareil compteur à gaz (100, 101), et
- au moins un module d'extension (3, 3,1, 20, 21, 36, 40, 41,1, 41,2, 50, 65, 77),
dans lequel l'appareil compteur à gaz (100, 101) comprend
- une partie de base (1, 30, 31),
- au moins un capteur de gaz (34, 44),
- une unité d'évaluation (26) traitant des signaux, et
- au moins un appareil de lecture (17, 17,1, ..., 17,4, 17,5),
dans lequel le ou chaque capteur de gaz (34, 44) est configuré pour mesurer respectivement au moins une grandeur de détection qui est en corrélation avec la concentration d'au moins un gaz cible,
dans lequel l'unité d'évaluation (26) est configurée pour
- déterminer la concentration respective d'au moins un gaz cible ou la concentration additionnée de plusieurs gaz cibles, et
- utiliser, pour la ou chaque détermination de la concentration d'un gaz cible, au moins une valeur mesurée de la ou d'une grandeur de détection,
dans lequel le ou chaque module d'extension (3, 3,1, 20, 21, 36, 40, 41,1, 41,2, 50, 65, 77) peut être relié de manière amovible à la partie de base (1, 30, 31),
dans lequel le ou au moins un module d'extension (3, 3,1, 20, 21, 36, 40, 41,1, 41,2, 50, 65, 77) comprend une mémoire de données de module d'extension (18, 18,1, 19, 24, 67) dans laquelle est enregistrée une identification du module d'extension (3, 3,1, 20, 21, 36, 40, 41,1, 41,2, 50, 65, 77),
dans lequel le ou chaque appareil de lecture (17, 17,1, ..., 17,4, 17,5) est configuré pour, lorsque le ou un module d'extension (3, 3,1, 20, 21, 36, 40, 41,1, 41,2, 50, 65, 77) est relié à la partie de base (1, 30, 31), lire sans contact la mémoire de données de module d'extension (18, 18,1, 19, 24, 67) de ce module d'extension (3, 3,1, 20, 21, 36, 40, 41,1, 41,2, 50, 65, 77),
dans lequel l'appareil compteur à gaz (100, 101) est configuré pour déterminer, en fonction d'un signal du ou d'un appareil de lecture, si le ou un module d'extension (3, 3,1, 20, 21, 36, 40, 41,1, 41,2, 50, 65, 77), qui comprend une mémoire de données de module d'extension (18, 18,1, 19, 24, 67), est relié ou non à la partie de base (1, 30, 31),
dans lequel l'appareil compteur à gaz (100, 101) est en outre configuré pour, lorsqu'un tel module d'extension (3, 3,1, 20, 21, 36, 40, 41,1, 41,2, 50, 65, 77) est relié à la partie de base (1, 30, 31),
- déterminer l'identification enregistrée et
- déclencher une opération en fonction de la présence et/ou du type de l'identification enregistrée et déterminée dans la mémoire de données de module d'extension (18, 18,1, 19, 24, 67),
**caractérisé en ce que**
le ou au moins un module d'extension (3) modifie une dépendance de la grandeur de détection par rapport à la concentration de gaz cible et/ou influence la détermination de la concentration de gaz cible en fonction de la grandeur de détection,
dans lequel l'appareil compteur à gaz (100, 101) comprend une mémoire de données d'évaluation (27),
dans lequel sont enregistrées une première relation fonctionnelle et au moins une autre relation fonctionnelle entre la grandeur de détection et la concentration de gaz cible,
dans lequel l'unité d'évaluation (26) présente, au moins temporairement, un accès en lecture à la mémoire de données d'évaluation (27) et
dans lequel l'unité d'évaluation (26) est configurée pour
- lorsque ce module d'extension (3) est relié à la partie de base (1, 30, 31), appliquer la première relation fonctionnelle à une valeur mesurée de la ou d'une grandeur de détection, et
- lorsque ce module d'extension (3) n'est pas relié à la partie de base (1, 30, 31), appliquer la ou une autre relation fonctionnelle à la valeur mesurée de cette grandeur de détection.

2. Agencement (110) selon la revendication 1,
**caractérisé en ce que**
le ou un module d'extension comprend un capteur pour une condition environnementale et
dans la première relation fonctionnelle, cette condition environnementale est prise en compte, tandis que dans l'autre relation fonctionnelle, une valeur par défaut est utilisée pour cette condition environnementale.

3. Agencement (110) selon l'une des revendications précédentes,
**caractérisé en ce que**
le ou au moins un module d'extension (50) comprend un autre capteur de gaz (34),
dans lequel l'autre capteur de gaz (34), qui fait partie intégrante de ce module d'extension (50), est configuré pour mesurer une grandeur de détection qui est en corrélation avec la concentration d'un gaz cible prédéfini.

4. Agencement (110) selon l'une des revendications précédentes,
**caractérisé en ce que**
le ou au moins un module d'extension (65) comprend une unité d'alimentation en tension externe (68),
dans lequel l'appareil compteur à gaz (100, 101) est configuré pour, lorsque ce module d'extension (65) est relié à la partie de base (1, 30, 31),
alimenter au moins un, de préférence chaque, consommateur électrique (61, 26) de l'appareil compteur à gaz (100, 101) à partir de l'unité d'alimentation en tension externe (68).

5. Agencement (110) selon la revendication 4,
**caractérisé en ce que**
l'appareil compteur à gaz (100, 101) comprend une unité d'alimentation en tension interne (66),
dans lequel l'appareil compteur à gaz (100, 101) est configuré pour,
- lorsque ce module d'extension (65) est relié à la partie de base (1, 30, 31), alimenter le ou au moins un consommateur électrique (61, 26) à partir de l'unité d'alimentation en tension externe (68) et
- sinon, à partir de l'unité d'alimentation en tension interne (66).

6. Procédé de configuration permettant de configurer un appareil compteur à gaz (100, 101),
dans lequel l'appareil compteur à gaz (100, 101) comprend
- une partie de base (1, 30, 31),
- au moins un capteur de gaz (34, 44),
- une unité d'évaluation (26) traitant des signaux, et
- au moins un appareil de lecture (17, 17,1, ..., 17,4, 17,5),
dans lequel le ou chaque capteur de gaz (34, 44) est configuré pour mesurer respectivement au moins une grandeur de détection qui est en corrélation avec la concentration d'au moins un gaz cible,
dans lequel l'unité d'évaluation (26) est configurée pour
- déterminer la concentration d'au moins un gaz cible ou la concentration additionnée de plusieurs gaz cibles et
- utiliser, pour la ou chaque détermination de la concentration d'un gaz cible, au moins une valeur mesurée de la ou d'une grandeur de détection,
dans lequel au moins un module d'extension (3, 3,1, 20, 21, 36, 40, 41,1, 41,2, 50, 65, 77) est fourni,
dans lequel le ou chaque module d'extension (3, 3,1, 20, 21, 36, 40, 41,1, 41,2, 50, 65, 77) fourni
- peut être relié de manière amovible à la partie de base (1, 30, 31) et
- comprend une mémoire de données de module d'extension (18, 18,1, 19, 24, 67), dans laquelle est enregistrée une identification du module d'extension (3, 3,1, 20, 21, 36, 40, 41,1, 41,2, 50, 65, 77),
dans lequel le procédé de configuration comprend les étapes selon lesquelles
un module d'extension (3, 3,1, 20, 21, 36, 40, 41,1, 41,2, 50, 65, 77) comportant une mémoire de données de module d'extension (18, 18,1, 19, 24, 67) est relié de manière amovible à la partie de base (1, 30, 31),
le ou un appareil de lecture (17, 17,1, ..., 17,4, 17,5) lit sans contact la mémoire de données de module d'extension (18, 18,1, 19, 24, 67) de ce module d'extension (3, 3,1, 20, 21, 36, 40, 41,1, 41,2, 50, 65, 77) et
l'appareil compteur à gaz (100, 101)
- établit, en fonction d'un signal provenant de l'appareil de lecture (17, 17,1, ..., 17,4, 17,5), que le module d'extension est relié à la partie de base (1, 30, 31),
- détermine l'identification enregistrée dans la mémoire de données de module d'extension (18, 18,1, 19, 24, 67) et
- déclenche une opération en fonction de la présence et/ou du type de l'identification enregistrée et déterminée dans la mémoire de données de module d'extension (18, 18,1, 19, 24, 67),
**caractérisé en ce que**
le ou au moins un module d'extension (3) modifie une dépendance de la grandeur de détection par rapport à la concentration de gaz cible et/ou influence la détermination de la concentration de gaz cible en fonction de la grandeur de détection,
dans lequel l'appareil compteur à gaz (100, 101) comprend une mémoire de données d'évaluation (27),
dans lequel sont enregistrées une première relation fonctionnelle et au moins une autre relation fonctionnelle entre la grandeur de détection et la concentration de gaz cible,
dans lequel l'unité d'évaluation (26) présente, au moins temporairement, un accès en lecture à la mémoire de données d'évaluation (27) et
dans lequel l'opération comprend l'étape selon laquelle l'unité d'évaluation (26)
- lorsque ce module d'extension (3) est relié à la partie de base (1, 30, 31), applique la première relation fonctionnelle à une valeur mesurée de la ou d'une grandeur de détection, et
- lorsque ce module d'extension (3) n'est pas relié à la partie de base (1, 30, 31), applique la ou une autre relation fonctionnelle à la valeur mesurée de cette grandeur de détection.
